# EUROPEAN PATENT SPECIFICATION

(11) **EP 1 066 369 B1**
(45) Date of publication and mention of the grant of the patent: **19.07.2006**
(21) Application number: 99912674.1
(22) Date of filing: 18.03.1999
(51) Int. Cl.: C12N 5/10, G01N 33/53, C12Q 1/68, C12N 15/10

(54) **SELECTION SUBTRACTION APPROACH TO GENE IDENTIFICATION**
SELEKTION/SUBTRAKTIONSANSATZ ZUR GENIDENTIFIZIERUNG
METHODE DE SELECTION-SOUSTRACTION UTILISEE DANS L'IDENTIFICATION DE GENES

(30) Priority: 18.03.1998 US 78432 P
(43) Date of publication of application: 10.01.2001
(73) Proprietor: Quark Biotech, Inc., Pleasanton, CA 94566 (US); THE BOARD OF TRUSTEES OF THE UNIVERSITY OF ILLINOIS, Urbana, Illinois 61801 (US)
(72) Inventor: GUDKOV, Andrei, Glencoe, IL 60022 (US); KONDRATOV, Roman, Chicago, IL 60607 (US)
(74) Representative: Ratcliffe, Susan Margaret
(86) International application number: PCT/US1999/005944
(87) International publication number: WO 1999/047643

(56) References cited:
- WO-A-96/12014
- US-A- 5 427 908
- US-A- 5 580 717
- SHOEMAKER D D ET AL: "QUANTITATIVE PHENOTYPIC ANALYSIS OF YEAST DELETION MUTANTS USING A HIGHLY PARALLEL MOLECULAR BAR-CODING STRATEGY" NATURE GENETICS, NEW YORK, NY, US, vol. 14, no. 4, 1 December 1996 (1996-12-01), pages 450-456, XP002043431 ISSN: 1061-4036
- HENSEL M ET AL: "SIMULTANEOUS IDENTIFICATION OF BACTERIAL VIRULENCE GENES BY NEGATIVE SELECTION" SCIENCE, AMERICAN ASSOCIATION FOR THE ADVANCEMENT OF SCIENCE,, US, vol. 269, 21 July 1995 (1995-07-21), pages 400-403, XP000645478 ISSN: 0036-8075 -& WO 96/17951 A (RPMS TECHNOLOGY LTD ; HOLDEN DAVID W (GB)) 13 June 1996 (1996-06-13)
- SINGHI AATUR D ET AL: "Selection-subtraction approach (SSA): A universal genetic screening technique that enables negative selection" PROCEEDINGS OF THE NATIONAL ACADEMY OF SCIENCES OF THE UNITED STATES OF AMERICA, vol. 101, no. 25, 22 June 2004 (2004-06-22), pages 9327-9332, XP002299313 ISSN: 0027-8424
- SAGERSTROEM C G ET AL: "SUBTRACTIVE CLONING: PAST, PRESENT, AND FUTURE" ANNUAL REVIEW OF BIOCHEMISTRY, PALTO ALTO, CA, US, vol. 66, 1997, pages 751-783, XP000990144 ISSN: 0066-4154
- MAHAN M J ET AL: "SELECTION OF BACTERIAL VIRULENCE GENES THAT ARE SPECIFICALLY INDUCED IN HOST TISSUES" SCIENCE, AMERICAN ASSOCIATION FOR THE ADVANCEMENT OF SCIENCE,, US, vol. 259, 29 January 1993 (1993-01-29), pages 686-688, XP001026153 ISSN: 0036-8075
- DIATCHENKO L. et al., "Suppression Subtractive Hybridization: A Method for Generating Differentially Regulated or Tissue-Specific cDNA Probes and Libraries", PROC. NATL. ACAD. SCI. USA, June 1996, Vol. 93, pages 6025-6030, XP002917474
- CRAMPTON J. et al., "The Isolation of Cloned cDNA Sequences Which are Differentially Expressed in Human Lymphocytes and Fibroblasts", NUCLEIC ACID RESEARCH, 1980, Vol. 8, No. 5, pages 6007-6017, XP002917475

### SEQUENCE LISTING

<110> Quark Biotech, Inc.
   The Board Of Trustees Of The University Of Illinois
<120> SELECTION SUBTRACTION APPROACH TO GENE INDENTIFICATION
<130> P450257EP
<140> 99912674.1
   <141> 1999-03-18
<150> US60/078432
   <151> 1998-03-18
<160> 6
<170> Patent In Ver. 2.1
<210> 1
   <211> 24
   <212> DNA
   <213> Artificial Sequence
<220>
   <223> Description of Artificial Sequence: primer
<400> 1
   atgaattcta ccgcggtggc atgg 24
<210> 2
   <211> 20
   <212> DNA
   <213> Artificial Sequence
<220>
   <223> Description of Artificial Sequence: primer
<400> 2
   atggatccag ttcctcggtc 20
<210> 3
   <211> 21
   <212> DNA
   <213> Artificial Sequence
<220>
   <223> Description of Artificial Sequence: primer
<400> 3
   ggctaggcca ttaaggccac c 21
<210> 4
   <211> 19
   <212> DNA
   <213> Artificial Sequence
<220>
<223> Description of Artificial Sequence: primer
<400> 4
   ggatccggcc gaggcggcc 19
<210> 5
   <211> 19
   <212> DNA
   <213> Artificial Sequence
<220>
   <223> Description of Artificial Sequence: primer
<400> 5
   ccttgaacct cctcgttcg 19
<210> 6
   <211> 24
   <212> DNA
   <213> Artificial Sequence
<220>
   <223> Description of Artificial Sequence: primer
<400> 6
   ttacttaagc tagcttgcca aacc 24

## Description

### CROSS REFERENCE TO RELATED APPLICATIONS

This application is a conversion of a United States Provisional Application filed March 18, 1998, Serial No. 60/078,432.

### BACKGROUND OF THE INVENTION

### TECHNICAL FIELD

The present invention relates to the creation of "tagged" expression libraries, wherein each cDNA clone is marked by a unique TAG. The present invention further relates to the identification of genes using different methods of TAG-based selection-subtraction that identify genes associated with complete or partial growth suppression, with cell death or with any other selectable phenotype, by recognition and comparison of their tagged clones before and after selection. Furthermore, the present invention relates to the elaboration of novel subtraction techniques and a novel type of expression vector, a retrophage.

### BACKGROUND ART

There is no established and reliable technology available for functional isolation of growth suppressive genetic elements.
Labelled deletion strains have been used in determining the biological functions of open reading frames of *S.cerevisiae (Nature Genetics,* NY, vol. 14, page 4, 1996 - Shoemaker D *et al*).
Mutants of *S.typhimurium* with attenuated virulence have been deleted by the use of DNA tags (*American Association for the Advancement of Science,* vol. 269, 1995 - Hensel *et* a*l*).
In WO 96/12014, again a general molecular tagging system is discussed. However, the aforementioned systems do not allow for the obtaining of expression libraries from complex libraries with each clone being tagged so they can be isolated. Method "SETGAP" (Pestov et al, 1994) is based on expression libraries but has the limitation of only identifying subsets of growth inhibitory clones that are strong enough to block DNA synthesis after induction and are not too toxic to kill the cells after expression induction. It would be useful to have a method that does not have these limitations.

Expression selection is a widely used method for identification and cloning of genes which involves the use of expression gene libraries. However, there is a problem with existing expression libraries in that they are significantly redundant. It would be useful to obtain expression libraries from complex libraries, in which each unique clone is tagged to facilitate isolation.

Functional identification of cytotoxic and cytostatic genetic elements is also an important and challenging task. Establishment of a reliable method allowing expression selection of such elements could greatly facilitate discovery of new tumor suppressor genes that are mutated, deleted or inactivated by other means in processes of tumor development and progression. On the other hand, cytotoxic genetic elements with certain cell specificity (for example, directed against cancer cells), may serve as efficient tools in gene therapy applications. Although there are powerful methods to identify positive regulators of cell growth, the approaches to isolation of toxic genes are limited to the laborious methods of genetic analysis based on karyotypic alterations in cancer cells and GSE and anti-sense RNA technologies.

The methods used with and the utility of the present invention can be shown by the following nonlimiting examples and accompanying figures.

### SUMMARY OF THE INVENTION

In accordance with the present invention, there is provided a method of creating expression libraries by marking desired clones with a unique tag. An expression library including individual clones marked with a unique tag sequence is also provided. Also provided is a method of isolating genetic elements associated with complete or partial cell growth suppression or cell death by isolating clones from an expression library which are lost in the cell population. A method of selection subtraction is provided by tagging a clone in an expression library, cloning the tagged clone into a vector, delivering the tagged clone to a target cell, and comparing tags before and after selection whereby toxic genes and the attached tags disappear.

### DESCRIPTION OF THE DRAWINGS

The advantages of the present invention will be readily appreciated as the same becomes better understood by reference to the following detailed description, when considered in connection with the accompanying drawings, wherein:
Figure 1 shows the general principle of selection-subtraction approach (SSA); during passaging, "toxic" cDNA clones disappear from the recipient cells transduced with cDNA expression library and are subsequently identified by subtraction;
Figure 2 shows the general principle of selection-subtraction approach based on the subtraction of individual TAGs that mark each library clone;
Figure 3 shows the structure of a novel type of expression vector combining the features of λ phage and retroviral vector, a retrophage;
Figure 4 shows the structure of adaptors used for the tagged library construction; a & b are adaptors for the tagged p53 fragmented cDNA library; c is an adaptor for tagged full-length cDNA library; d is a TAG-containing primer for full-length cDNA library;
Figure 5 shows the method of addition of TAGs to cDNA clones by PCR;
Figure 6 shows the method of addition of TAGs to cDNA clones during reverse transcription;
Figure 7a shows the structure of retroviral vector with incorporated T7 promoter sequences;
Figure 7b demonstrates the implication of this vector in a design of full-size tagged library construction; a is the structure of tag; b is the structure of a 5' adaptor; c is the structure of a 3' oligonucleotide used for first strand synthesis; d are the primers for TAG rescue/amplification;
Figure 8 presents the method for identification of differences in full-length library composition before and after selection, using hybridization to cDNA microarrays;
Figure 9 shows the TAG-based subtraction method using Mung Bean Nuclease treatment;
Figure 10 shows the TAG-based subtraction method using SSH;
Figure 11 describes the RNase H mediated subtraction method;
Figure 12 presents the scheme of construction of a randomly fragmented tagged p53 single gene library;
Figure 13 presents the general strategy of a tagged cDNA library (either single gene or total) construction and screening;
Figure 14 demonstrates the disappearance of "toxic" full-length p53 cDNA clone from transduced 041 and 10(1) cells during passaging, as detected by Southern blot analysis with the corresponding tag sequences;
Figure 15 presents the method of identification of "toxic" cDNA clones by arraying the library clones on nylon membrane and subsequent differential hybridization of these membranes to the pools of TAGs rescued from cells before and after selection (passaging);
Figure 16 presents the general strategy of tagged cDNA library (either single gene or total) construction and screening using the RNAse H-based approach;
Figure 17 demonstrates the results of RT-PCR from RNA before and after RNAse H-based tag subtraction procedure; bottom panel shows isolation of the inserts marked by differentially represented tags by PCR using vector sequences flanking the insert as primers;
Figure 18 illustrates technical steps of SSH-based procedure of tag subtraction; top panel shows polyacrylamide gel fractionation of the isolated tags treated with the indicated restriction enzymes; bottom panel represents an agarose gel with the products obtained after 40 cycles of PCR on the indicated templates;
Figure 19 demonstrates the efficacy and specificity of recovery of the library-derived inserts by RT-PCR using vector-specific primers;
Figure 20 presents PCR products of recovered inserts at different stages of selection of three retroviral cDNA libraries in three different cell types;
Figure 21 illustrates elimination of growth suppressive clone encoding p21/WAF1 sequence during passaging of target library-transduced cells monitored by Southern hybridization; and
Figure 22 presents Southern blot hybridization with the results of RNAse H-based subtraction procedure. Left panel shows elimination of 2A-specific tag during cell selection, while the right panel presents strong enrichment of this tag in RT-PCR product synthesized on RNA after subtraction with the tags isolated after selection.

### DETAILED DESCRIPTION OF THE INVENTION

The present invention provides an alternative method of identification of gene sequences whose expression in cells elicits growth suppression, apoptosis or any other selectable phenotype. It is based on the isolation of those particular clones from an expression library that are specifically lost in cell population transduced with the expression library under certain conditions of selection, i.e., simple passaging or sensitization to certain cytotoxic stimuli, by using gene subtraction procedures.

Standard molecular biology techniques known in the art and not specifically described are generally followed as in Sambrook et al., *Molecular Cloning: A Laboratory Manual,* Cold Springs Harbor Laboratory, New York (1989, 1992), and in Ausubel et al., *Current Protocols in Molecular Biology,* John Wiley and Sons, Baltimore, Maryland (1989). Additionally, standard methods in immunology known in the art and not specifically described are generally followed as in Stites et al. (eds), *Basic and Clinical Immunology (8*^{*th*} *Edition),* Appleton & Lange, Norwalk, CT (1994) and Mishell and Shiigi (eds), *Selected Methods in Cellular Immunology,* W.H. Freeman and Co., New York (1980).

Delivery systems (vectors) for delivering the expression cassette to the target cell are known in the art and a nonexhaustive list is provided herein below. Of particular interest are viral vectors as discussed below. Alternatively, a cell based system is used as a method of site-specific delivery (targeting) to the tumorogenic cells. The cells are generally selected from cells that shed and/or release the expression cassette to be incorporated by the tumorogenic cells as is known in the art. These cell are selected from cells that will "home" to the site of the tumorogenic cells. Alternatively, other methods know in the art for site specific delivery of cells are used to deliver the cells *in situ.*

Vectors which comprise the DNA encoding for a clone of the expression library are also provided by the present invention. The vectors can be constructed by those skilled in the art and should contain all expression elements necessary to achieve the desired transcription of the sequences. Other beneficial characteristics can also be contained within the vectors such as mechanisms for recovery of the nucleic acids in a different form. Phagemids are a specific example of such beneficial vectors because they can be used either as plasmids or as bacteriophage vectors. Examples of other vectors include viruses such as bacteriophages, baculoviruses and retroviruses, DNA viruses, cosmids, plasmids, liposomes and other recombination vectors. The vectors can also contain elements for use in either procaryotic or eucaryotic host systems. One of ordinary skill in he art will know which host systems are compatible with a particular vector.

The essential materials and experimental steps of the present approach are described below.

### 1. Expression Libraries

The approach is applicable to a wide variety of expression libraries including plasmid- and virus-derived expression systems in eukaryotes and prokaryotes. The source of genetic material may involve mixtures of cDNAs cloned in sense or anti-sense orientation, mixtures of fragments of genomic DNA or cDNA, fragments of individual cDNAs and mixtures of artificial sequences.

### 2. Library Delivery

Expression libraries are delivered to the test cells by an appropriate gene transfer techniques, including various methods of DNA transfection, infection by vector viruses or bacteriophages.

### 3. Selection Conditions

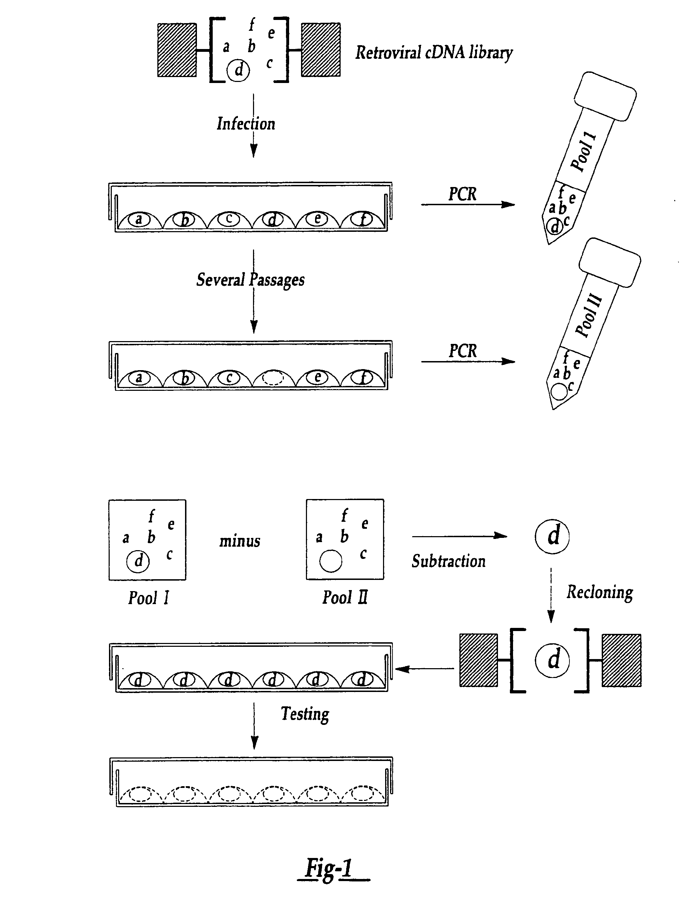

Growth suppressive clones will be gradually lost during propagation of the cell population carrying the library. Such clones may decrease growth rate or kill the cells under normal conditions of growth or they may be growth suppressive under specific conditions, including sensitization of cells to certain physical, chemical or biological treatments that have no or low effect on the majority of cells in library-carrying cell population. See Figure 1.

### 4. Screening Procedures

Expression library or the library inserts are rescued from the cells before and after selection. The rescue procedure is determined by the properties of the library and may be done by PCR on total cellular DNA, RT-PCR on cellular RNA, direct transformation of competent cells (episomal libraries) or virus rescue (libraries in retroviral, adenoviral or other viral vectors). The proportion of growth-suppressive sequences is expected to be decreased in the rescued libraries or library inserts after the selection. These sequences are isolated by using one of the gene subtraction techniques. See Figure 1.

Specifically, library-derived sequences of the resulting (rescued) mixture of molecules is subtracted from the original mixture of sequences either derived from the original library or rescued from the cells before selection. The complexity of the subtracted material is estimated. If the number of isolated clones is too large for individual testing, the material is subjected to a second round of selection. This procedure is repeated until the number of clones becomes low enough to allow individual testing. See Figure 1.

Technically, subtraction may be done in different ways, including manipulations with the sequences of the genes themselves and using a newly developed tagged library screening technique. During construction of cDNA libraries, each clone is marked with a short 80 bp random DNA fragment (TAG).

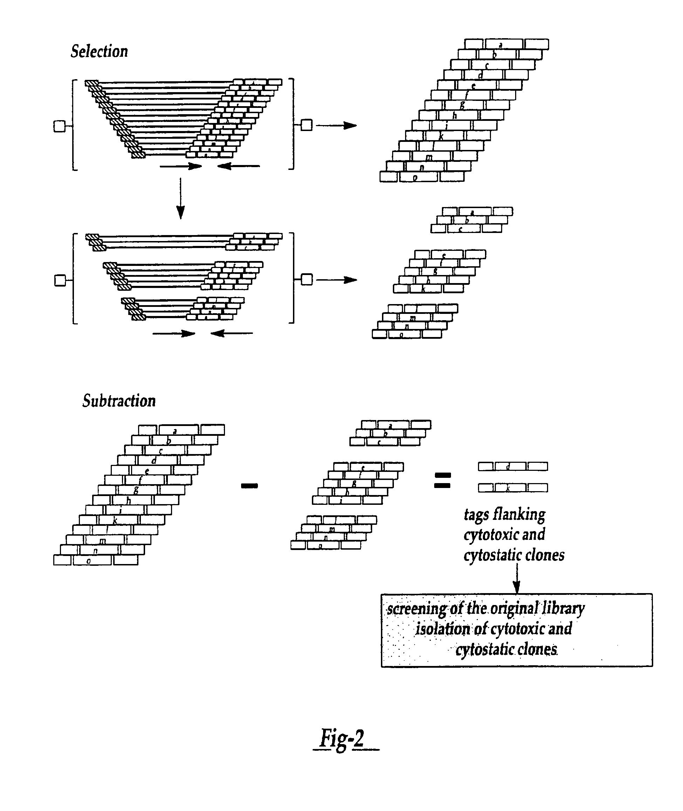

A tagged library is cloned in retroviral vector and delivered to target cells. Total cell RNA is isolated immediately after introduction into the cell and after application of selection conditions, as aforementioned. During selection, cells expressing "toxic" genes are eliminated from the population and TAGs of these "toxic" genes will disappear. Comparison is made of TAGs before and after selection, (for example, using PCR-based subtraction methods), and disappearing TAGs marking putative cytotoxic genes are isolated. These TAGs are then used as probes to identify the cytotoxic and cytostatic genetic elements which they mark in the library. (See Figure 2).

The use of TAGs has a number of advantages: (1) the TAGs have uniform size and are designed in such a way as to facilitate their isolation by PCR or other methods; (2) each clone in the library is marked by a unique TAG, with the full-length, truncated or anti-sense variants of the same gene marked with different TAGs. Subtraction allows identification of those particular clones that are biologically functional; and (3) this method can be applied for the detection of "toxic" sequences regardless of their origin since all subtraction procedures are done in standard conditions (full length cDNA, GSE, etc.).

### 5. Isolation of Growth-suppressive Genetic Elements with Cell-Specific Activity

To isolate genetic elements that are growth-suppressive for certain cell types (tumor cells, virus-infected cells, cells of certain tissue, etc.) that have no or low effect on other cell types, the selection procedure is applied in parallel to the target cells and to the cells of other types that are not supposed to be affected by the isolated elements (i.e., transformed cells and non-transformed cells or similar origin, etc.). The resulting mixtures of molecules obtained by selection subtraction approach (SSA) procedures are compared, to isolate those sequences that are specifically lost during selection of the target but not control cells. Specifically, such sequences are isolated by subtraction of the material obtained from control cells from the material obtained from the target cells. Different subtraction procedures can be applied for the isolation of the desired tags, including direct hybridization comparison of the populations of pre- and post-selected tags, RDA, SSH and any other methods allowing for the comparison of two populations of nucleic acid sequences.

### 6. Expected Growth Suppressive Genetic Elements and their Possible Applications

This method has two general applications: (I) isolation of growth suppressive molecules (DNA, RNA or polypeptides) with specificity against certain cell types; and (II) identification of new genes involved in negative growth regulation (i.e., tumor suppressive genes, genes involved in control of apoptosis, cellular senescence, sensitivity to chemical, physical or biological treatments, etc.).

### 7. Other Types of Selections of Biologically Active Genetic Elements

It is noteworthy that the range of applications of SSA is not limited to the isolation of cytotoxic or cytostatic elements. It can be used for isolation of genetic elements that induce any cell phenotype that can be used as a selective trait to enrich or exhaust cell population (i.e. expression of cell surface antigens, alterations in cell adhesion, cell size, etc.).

### GENERAL METHODS

General methods in molecular biology: Standard molecular biology techniques known in the art and not specifically described are generally followed as in Sambrook et al., *Molecular Cloning: A Laboratory Manual,* Cold Springs Harbor Laboratory, New York (1989, 1992), and in Ausubel et al., *Current Protocols in Molecular Biology,* John Wiley and Sons, Baltimore, Maryland (1989). Polymerase chain reaction (PCR) is carried out generally as in *PCR Protocols: A Guide to Methods and Applications,* Academic Press, San Diego, California (1990). Reactions and manipulations involving other nucleic acid techniques, unless stated otherwise, are performed as generally described in Sambrook et al., *Molecular Cloning: A Laboratory Manual,* Cold Springs Harbor Laboratory, New York (1989) and methodology as set forth in United States patents 4,666,828; 4, 683,202; 4,801,531; 5,192,659 and 5,272,057.

### SPECIFIC METHODS

### Expression libraries that can be used for SSA

The developed approach is applicable to a wide variety of expression libraries including plasmid- and virus-derived expression systems in eukaryotes and prokaryotes. The source of genetic material may involve mixtures of cDNAs cloned in sense or anti-sense orientation, mixtures of fragments of genomic DNA or cDNA, fragments of individual cDNAs and mixtures of artificial sequences. However, the use of conventional expression libraries for SSA could be complicated by redundancy of the libraries that might carry similar sequences in both functional and non-functional forms and by difficulties of rescuing and subtraction of the inserts of variable length. These obstacles could be overcome by the use of Tagged libraries.

### New "retrophage" expression vector

The phage lambda-based vectors have significant advantages over plasmid vectors in preparation and screening of complex libraries. The efficacy of ligation of the inserts into a phage vector depends significantly less on the insert size (within the regular range of lengths of the majority of cDNAs). Phages can be plated at much higher density than bacterial colonies thus increasing the efficiency of screening of large libraries. Hybridization screening of the prints of phage plaques on nylon or nitrocellulose is a far more sensitive and reliable technique than that of the bacterial colonies. Prints of plaques of phage libraries are highly reproducible and can be considered cDNA arrays allowing one to simultaneously analyze expression of numerous genes in a single hybridization.

At the same time, retroviral expression vectors have a series of advantages specifically valuable for generation and screening of expression libraries (cDNA, GSE, antisense RNA, etc.). They represent the most efficient delivery vehicle that brings the gene of choice into the cell and determines its integration and stable expression in the genome at different levels within the range of expression of cellular genes.

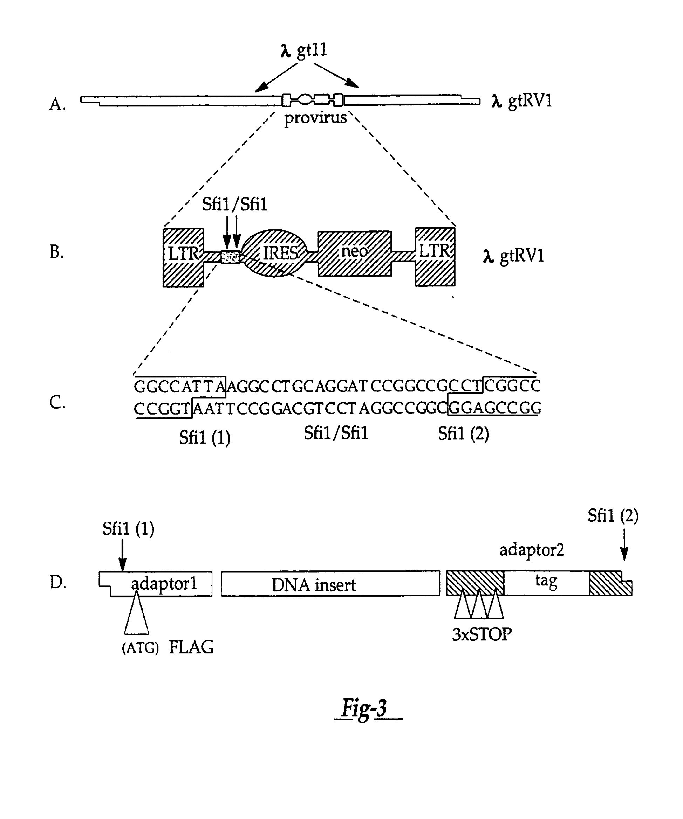

The advantages of phage and retroviral vectors were combined by creating a new retrovirus expression vector inside the phage DNA. The schematic presentation of this new vector is shown at the Figure 3. The new vector consists of the λgt11 phage with a LXSN retrovirus vector inside. The polylinker inside LXSN was substituted for two SfiI sites with different cohesive ends to make it possible for oriented cloning of the inserts and to avoid self-ligation of the two arms of the phage DNA. λgt11 can accept fragments of DNA up to 7.2 kb in length. The size of provirus part is about 3 kb. Thus, the cloning capacity of the new vector is up to 4 kb.

To prove the efficacy of the created vector, its ability to produce infectious retroviruses after transfection into packaging cells was directly compared with that of the LXSN plasmid vector and found it to be only 2 to 3 fold lower if calculated per microgram of DNA. The phage/retrovirus vector can be very useful for preparation of large expressing cDNA libraries with millions of recombinant clones. To increase the cloning capacity of the phage/virus vector we made a new modification was made to the original vector, named 1gtLXL, which lacks selectable marker gene and has cloning limit up to 5 kb.

### METHODS OF PREPARATION OF TAGGED LIBRARIES

### Nature of tags

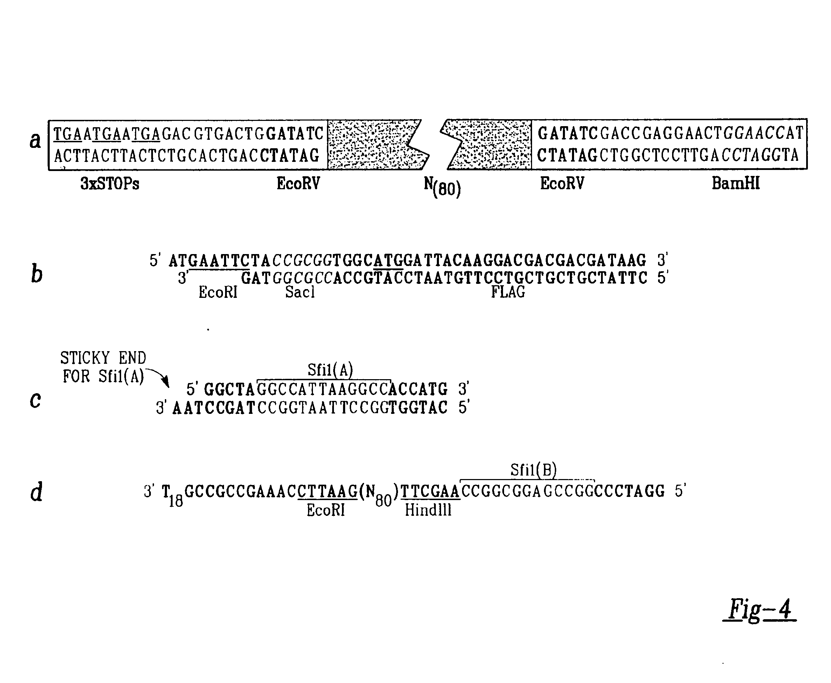

As tags one can use any highly divergent sequences, the complexity of which significantly exceeds the complexity of the library. For example, random oligonucleotides or fragments of low redundant genomes (i.e. DNA of prokaryotes) of a reasonable size range (10-1000 bp) can be used as tags. The choice of tags is dictated by the method of tag isolation, subtraction and identification (see below). For example, random oligonucleotides 80 bp long flanked by short (approximately 20-30 bp) sequences carrying the appropriate restriction sites and suitable for efficient PCR amplification (Figure 4) were used as tags. Tags are introduced in the library-carrying vectors at the sites at which they cannot interfere with the expression or delivery.

### Introduction of Tags into expression libraries

Oligonucleotides can be introduced into the library at different stages of library preparation including the following options:
1) Tags are introduced into the population of vector molecules prior to library preparation, and a highly diverged population of individually tagged vectors is used for cloning of inserts;
2) Tags are introduced in already prepared libraries by cloning the Tagging DNA fragments into the defined site of the existing libraries;
3) Tags are added to the inserts prior to cloning into expression vectors.

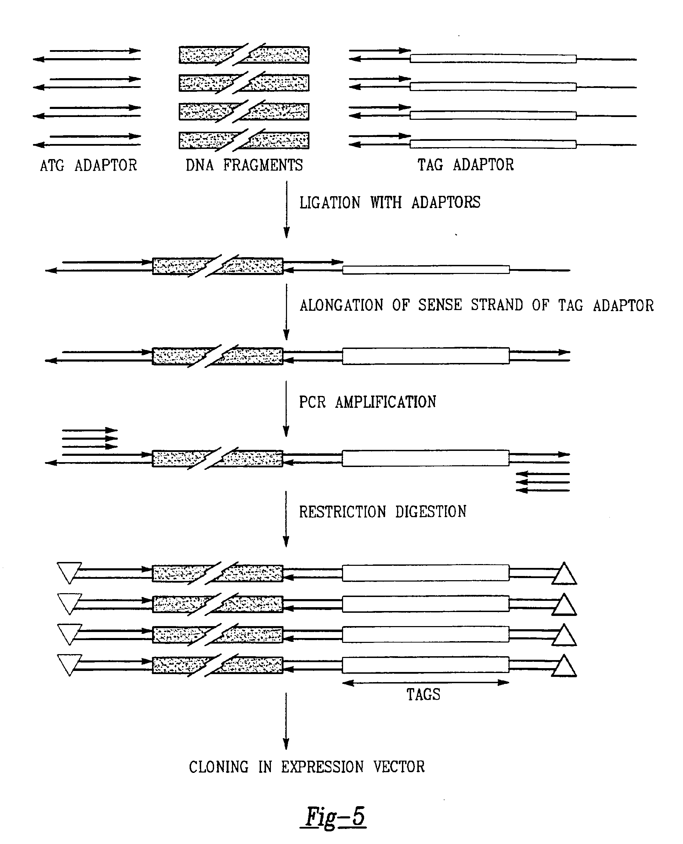

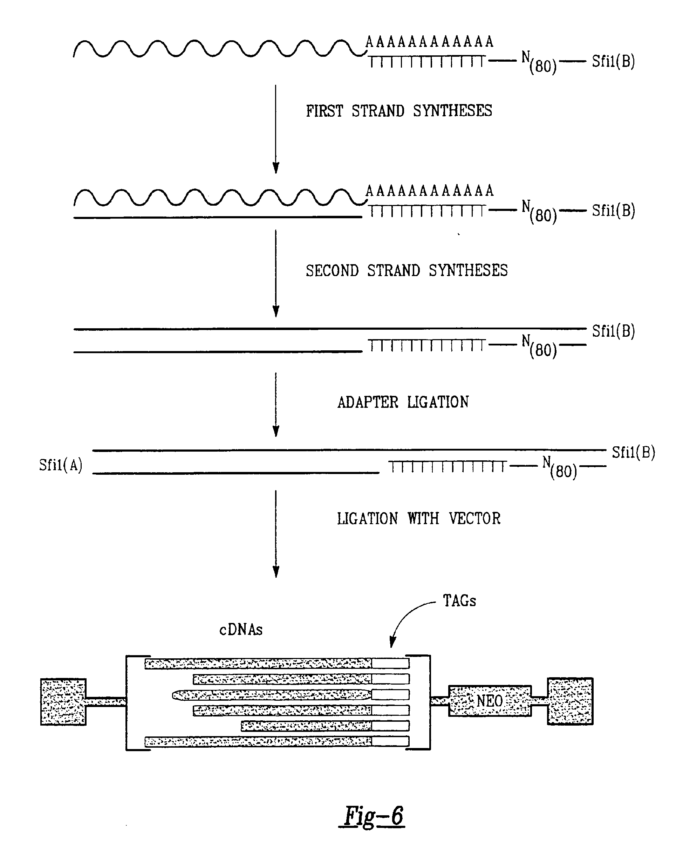

Among the three above options, the third one has clear advantages since it ensures that every individually generated clone is marked by a specific tag sequence. Two examples of Tagged library construction are shown below. In one of them, oligonucleotide tags are added to cDNA fragments by ligation followed by PCR amplification (Figure 5). Another example shows 80-nucleotide long Tags incorporation during the first stage of cDNA synthesis. In this method, Tag sequences are included as part of the primers used for reverse transcription (Figure 6).

### Library delivery and selection

The choice of methods for library delivery and selection depends on the nature of the expression vector, cell type and experimental needs. Plasmid libraries can be delivered to target cells by transfection, lipofection, electroporation and other available techniques. Viral vectors can be first delivered to packaging cells to convert the library from the plasmid form into the mixture of viral particles, which then is used for the infection of target cells. Examples of construction and delivery of retroviral libraries are shown below; methods of their delivery to target cells were described earlier (Gudkov & Roninson, 1996).

Selection of putative cytotoxic and cytostatic clones may involve simple propagation of library-transduced cell populations, thus allowing the cells to pass through several divisions to reduce the proportion of growth arrested or dead cells in the final population. Isolation of the clones that are not cytotoxic or cytostatic by themselves, but which display their growth suppressive activity under certain conditions of cell maintenance or treatment requires application of the appropriate selection. Such selection should be noncytotoxic or low-cytotoxic by itself and should not lead to a significant cell death or growth arrest that might jeopardize identification of the desired library clones cooperating with the applied treatment in facilitating cell death or growth inhibition. For example, genetic elements restoring the activity of the p53 pathway by blocking p53 inhibitors (i.e., papilloma virus protein E6 or cellular p53 inhibitor MDM2, see examples below) are expected to cause cell sensitization to DNA-damaging treatments since p53 plays a role of negative growth regulator that is activated under conditions of genotoxic stress.

It is noteworthy that the range of applications of SSA is not limited to the isolation of cytotoxic or cytostatic elements. In principle, it can be used for isolation of genetic elements that induce any cell phenotype that can be used as a selective trait to enrich or exhaust cell population (i.e. cell surface antigens, cell adhesion, cell size, etc.).

### METHODS OF IDENTIFICATION AND CLONING OF GROWTH SUPPRESSIVE CLONES

### A. Methods based on subtraction of the library inserts

SSA can be applied to regular untagged libraries. In this case, the inserts are isolated shortly after library delivery (before selection) and after selection and the populations of inserts are compared with each other to identified the inserts that were eliminated during selection.

### Recovery of the inserts

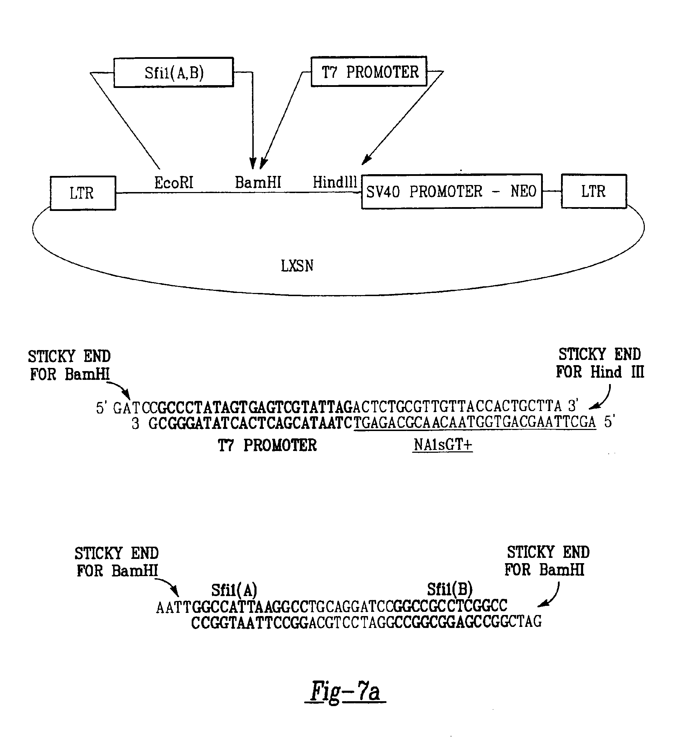

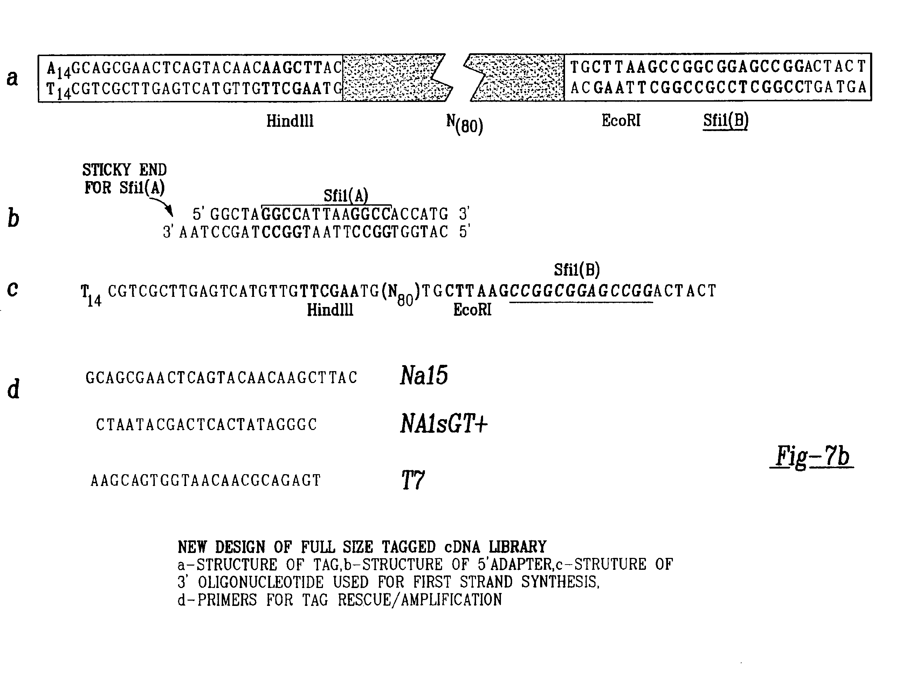

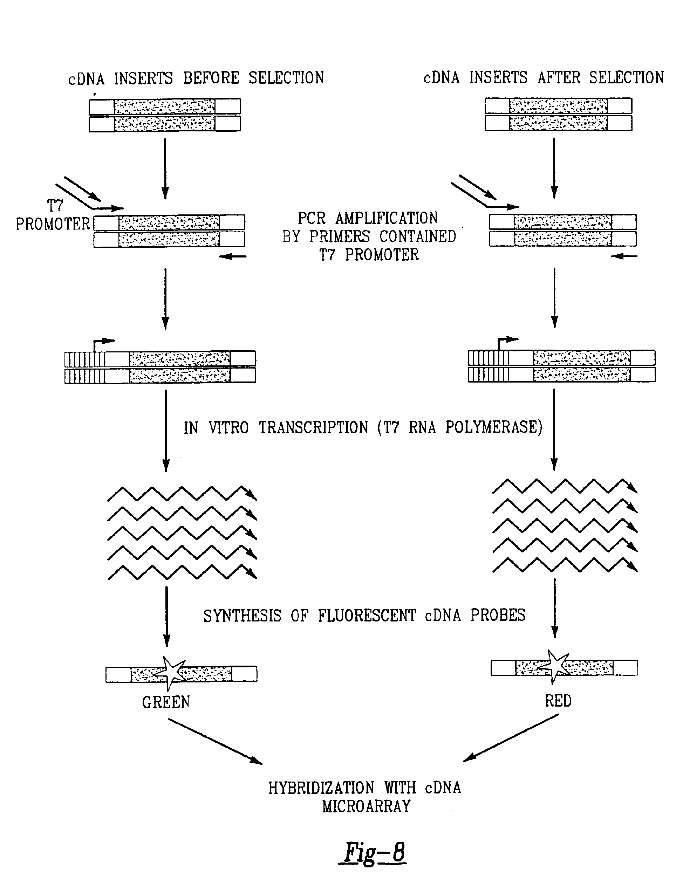

Inserts can be recovered from the cells by PCR, using sequences flanking the tags as primers. Either cellular DNA or RNA can be used as templates. In the latter case, cDNA should be synthesized first using oligo(dT), random oligonucleotides or specific oligonucleotides corresponding to vector sequences as primers. For the libraries constructed in episomal vectors or in the vectors allowing to excise the inserts by Cre Lox recombinase (Russ et al., 1996) transformation of competent bacterial cells by isolated plasmid DNA could be used instead of PCR. Example of the recovery of inserts from the cells transduced with retroviral libraries can be found in earlier published papers (Gudkov et al., 1994; Gudkov & Roninson, 1996). Rescuing of the inserts may involve the stage of amplification of the isolated sequences by RNA-polymerase reaction. To make it possible, the expression vectors used for the library preparation can be supplied with the appropriate sequences serving as promoters of efficient prokaryotic RNA polymerases (i.e., RNA polymerase 7, see Figures 7 and 8). RNA is synthesized using PCR products with T7 promoter as templates.

### Comparison of pools of inserts using hybridization with DNA microarrays

Comparison of the contents of two populations of inserts isolated from the cells before and after selection can be done using differential hybridization with microarrays containing, for example, a large number of cDNAs representing individual genes or the inserts from the library used for the screening. Hybridization probes are prepared from the two pools of DNA inserts for comparison (i.e., by labeling with two different fluorescent nucleotide precursors; labeling can be done in different ways, including random primed DNA-polymerase reaction or T7 RNA polymerase reaction) that are then used for hybridization with an appropriate DNA chip (Schena et al., 1995). Individual DNA probes showing differential hybridization are picked and used for further characterization.

### Isolation of the inserts eliminated during selection by the SSH technique

The comparison of the two pools of DNA inserts and isolation of differentially represented sequences can be done using a variety of gene subtraction protocols including previously described methods of RDA (representation differential analysis, Lysitsin et al., 1993) or SSH (suppression subtraction hybridization, Diatchenko et al., 1996). Both PCR-based methods require restriction digestion of the isolated inserts by frequently cutting enzymes, ligation with synthetic adaptors and PCR amplification. The resulting fragments of differentially represented inserts are then used as probes to clone their corresponding full size inserts.

### Identification of the differentially represented inserts by colony or plaque hybridization

An expression library in the form of a mixture of recombinant plasmids or phages is delivered to host bacterial cells. The prints of the resulting colonies or plaques on nitrocellulose or nylon are then prepared according to standard protocols and hybridized with the recovered inserts. Comparison of hybridization results obtained with pre- and post-selection inserts allows one to pick differentially represented clones for further characterization.

### B. Methods based on subtraction of Tags

A set of different gene subtraction procedures can be applied to identify and isolate differentially represented tags. These procedures involve both previously designed techniques, which have been adapted to the specific needs of tag subtraction, and novel approaches. Some of them are described below.

### Tag subtraction, using modified RDA technique and single stranded DNA-specific nuclease

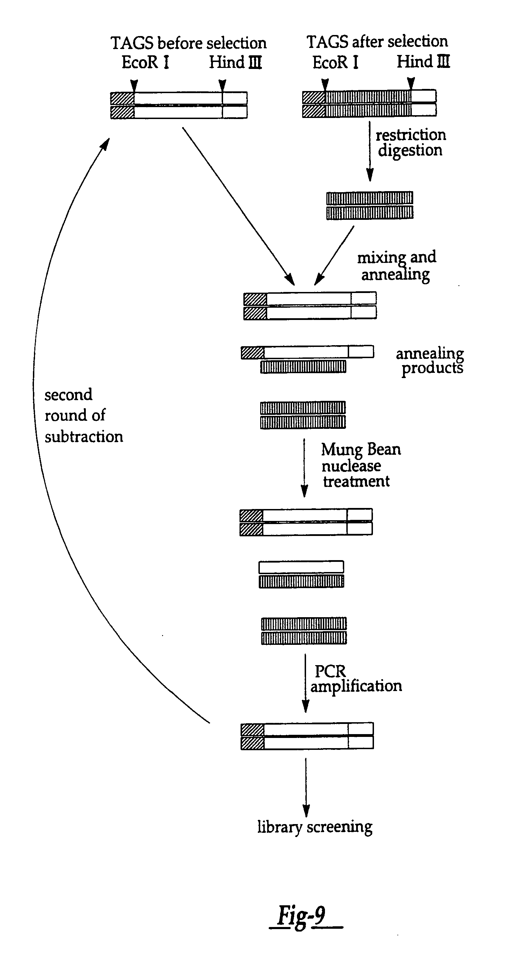

The principles of this approach are shown in Figure 9 For this method, tag sequences should be flanked by cleavage sites for restriction enzyme(s) (i.e., HindIII and EcoRI, as shown in Figures 4 and 8). Flanking sequences are removed from tags isolated from the cells after selection (TAG B) by digestion with the appropriate restriction enzymes and the fragments representing tag sequences are gel-purified. TAGs from the cells before selection (TAGs A) are also isolated and used uncut. An excess of TAGs B DNA is mixed with TAGs A, precipitated, dissolved in hybridization buffer and hybridized under standard conditions to reach saturated hybridization. The resulting mixture is diluted and treated with a nuclease specific for single-stranded DNA (i.e., Mung Bean nuclease) to remove non-annealed flanking sequences. DNA molecules that retained flanking adaptor sequences are then amplified by PCR. This procedure is repeated several times and the resulting material is used as a probe to isolate clones from the original library containing differentially represented inserts.

### Tag subtraction using modified SSH technique

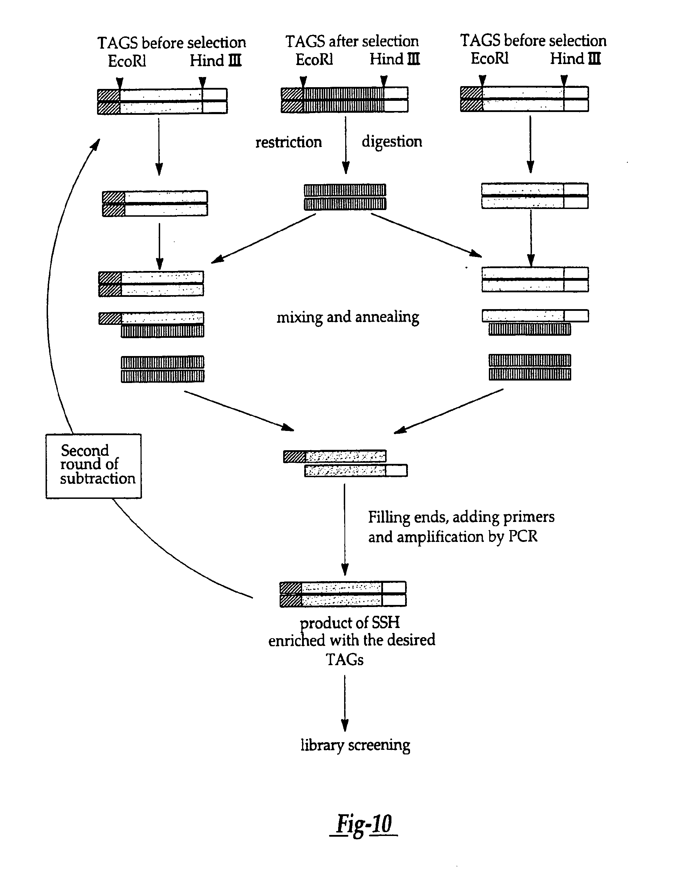

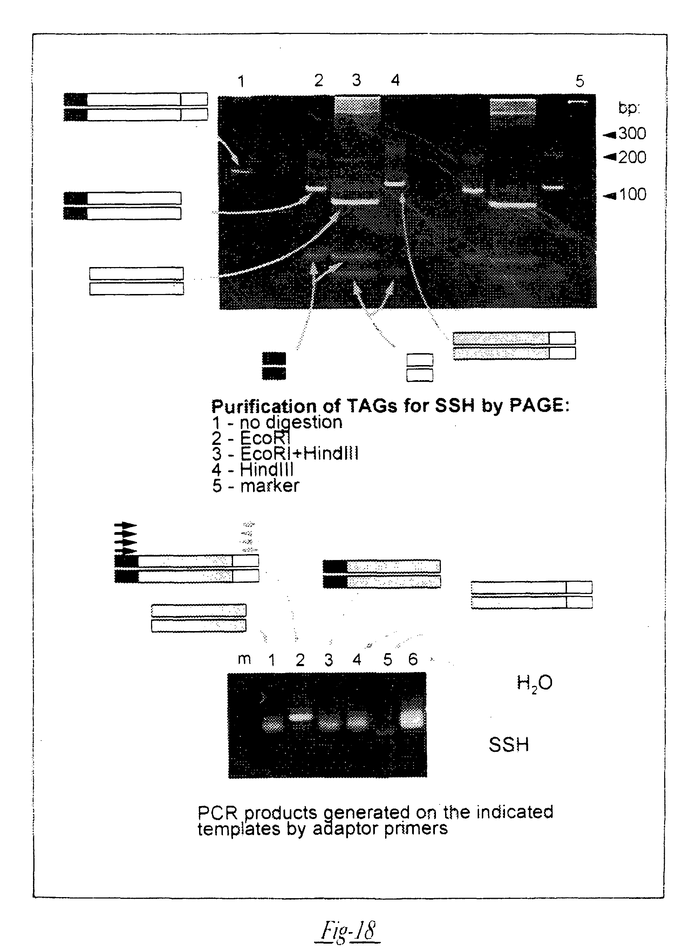

For this method, sequences that flank tags from the left and right sides should be different and should contain cleavage sites for two different restriction enzymes (i.e., HindIII and EcoRI, as shown in Figure 10). Driver DNA (TAGs B isolated from cells after selection) is cut with both enzymes and carefully gel-purified. Tester DNA (TAGs A, tags from the library prior to selection) is divided in two portions, which are cut with each of the restriction enzymes separately and also purified from undercut material. After that, a small amount of tester TAGs, of each type, are mixed with an excess of driver TAGs in two separate tubes, they are precipitated, dissolved in hybridization buffer, and then annealed at standard conditions. Then the hybridization mixtures from two separate tubes are combined together and further annealed. The resulting material is used for PCR. Only those sequences that are lost or underrepresented in the post-selection population of tags are preferentially amplified (Figure 18).

### RNAseH-based subtraction method

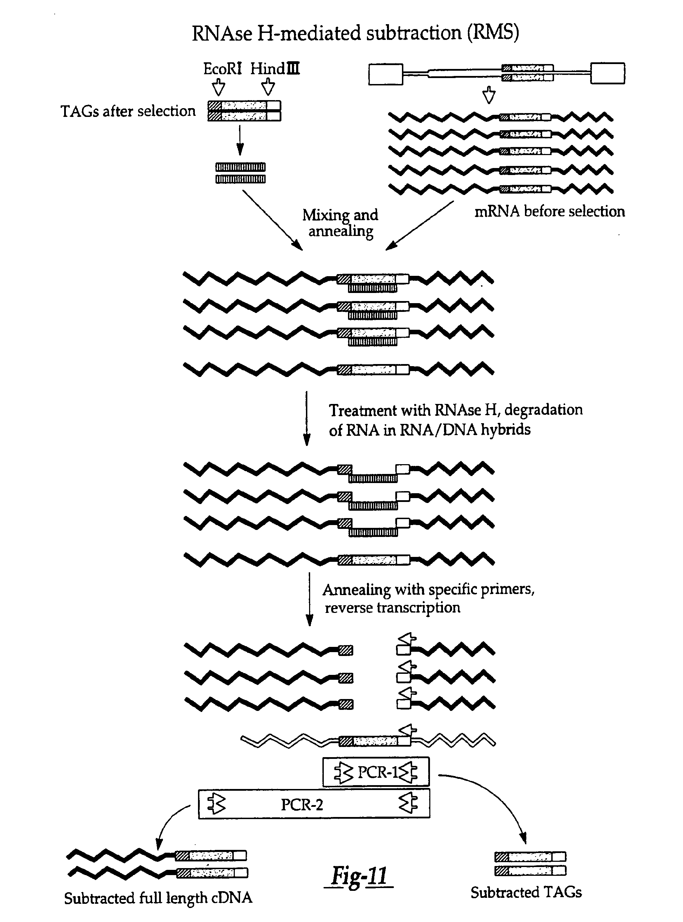

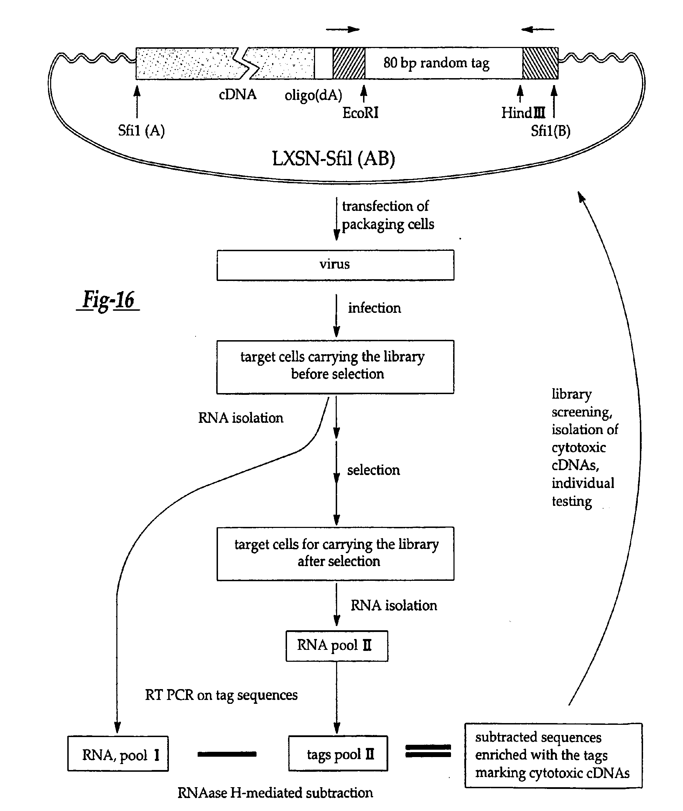

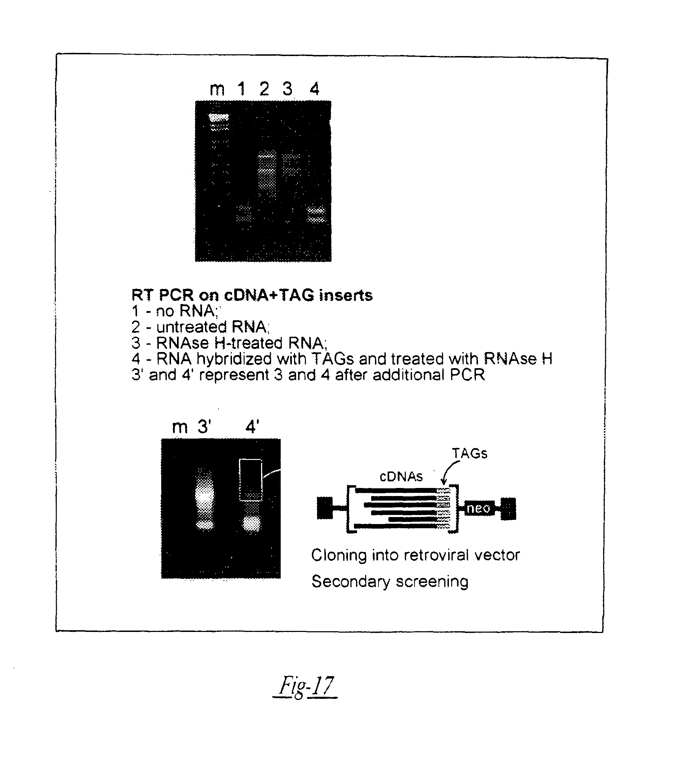

A new subtraction procedure was developed specifically for SSA technique that is based on the use of RNAse H for isolation of differentially represented sequences. The general principle of the method is illustrated by Figures 11 and 16. In this method, only tags from the post-selected library (TAGs B) are isolated by PCR. These tags are used as a tool to subtract from the original RNA population those sequences that have not been lost during selection. The mixture of TAGs B oligonucleotides isolated by PCR is digested by the appropriate restriction enzymes to remove uniform flanking adaptor-derived sequences and tag-related fragments are gel-purified. The resulting DNA is denatured, mixed with RNA isolated from the cells prior to selection, dissolved in hybridization buffer and annealed under standard hybridization conditions of high stringency. The resulting mixture of RNA, DNA and RNA:DNA hybrids is then dissolved in an appropriate buffer and treated with RNAse that destroys RNA in RNA/DNA hybrids. Reverse transcription is then done on the remaining RNA using vector-specific antisense primers to specifically copy library-derived transcripts. The remaining tags are then amplified by PCR using flanking vector or adaptor sequences as primers. Isolated tags are used as hybridization probes for detection of their corresponding clones from the original library. This subtraction procedure allows one to directly synthesize a full size inserts by using as sense primers vector sequences located upstream of the insert. In this case, the resulting material is directly cloned into expression vector and tested for biological activity (Figure 17).

It should be noted that a similar procedure can be applied to isolate differentially represented inserts from regular untagged expression libraries.

### Identification of differentially represented tags by colony or plaque hybridization

An expression library in the form of a mixture of recombinant plasmids or phages is delivered to host bacterial cells. The prints of the resulting colonies or plaques on nitrocellulose or nylon are then prepared according to standard protocols and hybridized with the recovered tags. Comparison of hybridization results obtained with pre- and post-selection tags allows one to pick differentially represented tags for further use as probes to isolate their corresponding library clones by conventional hybridization screening procedures.

### Isolation of the inserts based on the results of tag subtraction

There are two main possibilities for isolating library clones containing identified tags. One involves the use of tags as hybridization probes to screen the original library by conventional colony- or plaque-hybridization. The other approach is based on PCR, using tag-derived antisense primer and vector-derived sense primer corresponding to the sequence upstream of the inserts.

### Secondary screening procedures

A single round of SSA may not necessarily result in isolation of a pure set of tags or inserts that are differentially represented in two pools of DNA (prior and after selection); the mixture obtained after the first round may be enriched with the desired sequences but could also be significantly contaminated with the sequences equally represented in both DNA samples. To reach better purification, the material obtained after the first round will be used to isolate the corresponding clones from the original library and the resulting sublibrary will be subjected to a second round of selection instead of carrying out individual characterization of the isolated inserts after the first round.

Isolation of clones that are specifically toxic for certain cell type may require additional selection of the isolated mixture of clones in the cell type of different origin in order to define those clones that have selective biological activity against the cells of choice.

### Testing of biological activity of recombinant clones isolated by SSA

Recombinant clones isolated by SSA will be individually tested for their biological activity. They will be delivered by transfection or infection into target cells and the proliferation of transduced cells will be monitored under conditions used for the selection. Vectors carrying biologically inactive inserts or already known cytotoxic or cytostatic genetic elements (i.e. 2A gene of poliovirus or p21/wafl gene that block cap-dependent translation and induce G1 arrest, respectively) will be used as controls for comparison. Relative efficiency of delivery of different constructs will be controlled (i.e. by co-transfection with the LacZ- or GFP-expressing constructs). The behavior of the transduced cells will be monitored using either GFP fluorescence or selection in the presence of the appropriate selecting agent (G418, hygromycin, puromycin, phleomycin, etc., depending on the vector). Clones with the confirmed biological activity will be picked for further detailed analysis.

### EXAMPLES

### Example 1. Construction of tagged retroviral libraries expressing fragments of individual cDNAs

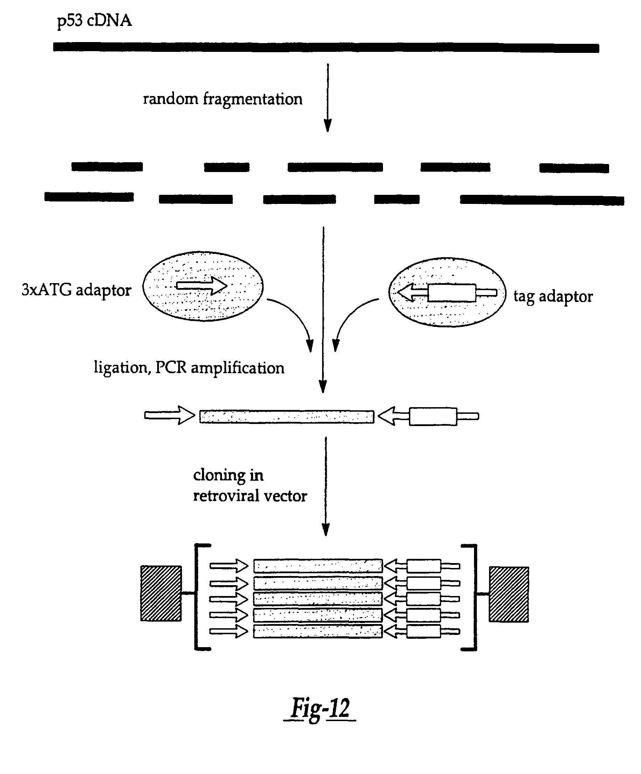

cDNA inserts were gel-purified and partially digested by DNAse I in the presence of Mn⁺⁺ (1µg DNA with 0.01 U of DNAse I in 10mM MnCl₂, 50mM Tris-HCl at 16°C at different time points (between 1 and 5 min)). The ends were filled in by a mixture of Klenow fragment and T4 DNA polymerase, and the cDNA fragments ranging in size between 200 bp and full-length cDNA were ligated with two synthetic adaptors, one encoding FLAG peptide in-frame with the ATG codon in Kozak's consensus, and containing EcoRI site and the other containing three stop codons in all reading frames followed by random 80 nucleotides (tag), flanked by EcoRV sites inside short "arms'' for future PCR amplification, and BamHI site for cloning (see Figure 4). The ligated material was amplified by PCR (10 cycles) with the following primers: sense - ATGAATTCTACCGCGGTGGCATGG (specific for 5' adapter ) and antisense - ATGGATCCAGTTCCTCGGTC ( specific for 3' adapter). PCR products were fractionated and gel-purified from 2% agarose, cut with BamHI+EcoRI restriction endonucleases and ligated into the LXSN vector. The complexity of the resulting libraries was approximately 30-40x10⁶ individual clones. The size of the inserts and proportion of recombinant clones were checked by PCR using sense and antisense primers corresponding to flanking vector sequences (Figure 12).

Fragmented retroviral libraries of similar characteristics were constructed from cDNAs for human p53, ATM, MDM2 and E6-AP genes. For example, the library of p53 fragments contained inserts between 250 and 1300 bp (total length of two adapters is 170 bp, total length of the full size of the coding region of p53 cDNA - 1200 bp). More than 95% of the clones contained inserts.

### Example 2. Construction of tagged retroviral cDNA libraries

Two retroviral tagged libraries of mouse embryonic cDNA were constructed, using a mixture of poly(A) RNA isolated from 7, 11, 14, 17 day-old mouse embryos. Figure 6 shows the structure of oligonucleotide which was used as primers for cDNA synthesis for the first library. This oligonucleotide contains 18 dT residues in its 3' end, EcoRI and HindIII sites (to cut TAGs' arms), 80 bp random fragment (TAG itself) and SfiI(B) site for cloning. A library was constructed with Gibco BRL cDNA synthesis kit, 5' adapter contained ATG codon and SfiI (A) site for cloning. Such design of inserts allowed oriented cloning to be done using a single restriction enzyme. The library was cloned in LXSN SfiI retroviral vector (LXSN with modify MCS). Frequency and size of inserts was checked by PCR using sense GGCTAGGCCATTAAGGCCACC (specific for 5' adapter), and antisense GGATCCGGCCGAGGCGGCC (specific for 3' TAGs "arm") primers. Library containing 97% of recombinant clones with the inserts ranging between 500-2000 bp (the total size 3' and 5' adapters was 170 bp). The total complexity of the library is approximately 1,000,000 individual clones. This library was prepared as independent separate batches containing 30-40 thousand clones in each. The below-described experiments were done using two randomly picked batches.

Another library from the same starting RNA was constructed using similar procedures with the following modifications introduced to improve the efficacy of recovery of tags and inserts. New tagging oligonucleotide was used containing 14 dT residues at its 3'-end, a new flanking sequence for efficient PCR amplification, the stretch of 80 random nucleotides flanked by EcoRI/HindIII sites and SfiI (B) site for cloning. 5' adapter was identical to that used for the above-described library. The library was cloned in pLXSN vector with a modified sequence around cloning site (see Figure 7a,b). This modification involves introduction of double SfiI site for directed cloning followed by T7 promoter in antisense orientation and 20 bp sequence termed Na1GT+ that is known as being highly efficient as a PCR primer (Luda Diatchenko, personal communication). This library has similar complexity and proportion of recombinant clones.

### Example 3. New retroviral ("retrophage") vectors for generation and efficient screening of expression libraries

The phage lambda-based vectors have significant advantages over plasmid vectors in preparation and screening of complex libraries. The efficacy of ligation of the inserts into phage vector significantly less depends on the size of insert (within the regular range of lengths of the majority of cDNAs). Phages can be plated at much higher density than bacterial colonies thus increasing the efficiency of screening of large libraries. Hybridization screening of the prints of phage plaques on nylon or nitrocellulose is a far more sensitive and reliable technique than that of the bacterial colonies. Prints of plaques of phage libraries are highly reproducible and can be considered cDNA arrays allowing one to simultaneously analyze expression of numerous genes in a single hybridization.

At the same time, retroviral expression vectors have a series of advantages specifically valuable for generation and screening of expression libraries (cDNA, GSE, antisense RNA, etc.). They represent the most efficient delivery vehicle that brings the gene of choice into the cell and determines its integration and stable expression in the genome at different levels within the range of expression of cellular genes.

The advantages of phage and retroviral vectors were combined by creating a new retrovirus expression vector inside the phage DNA. The schematic presentation of this new vector is shown at the Figure 3. The new vector consists of the λgt11 phage with LXSN retrovirus vector inside. The polylinker inside LXSN was substituted for two SfiI sites with different cohesive ends to make it possible oriented cloning of the inserts and to avoid self-ligation of the two arms of the phage DNA. λgt11 can accept the fragments of DNA up to 7.2 kb in length. The size of provirus part is about 3 kb. Thus, the cloning capacity of the new vector is up to 4 kb.

To prove the efficacy of the created vector, its ability was directly compared to produce infectious retroviruses after transfection into packaging cells with that of the LXSN plasmid vector and found it to be only 2 to 3 fold lower if calculated per microgram of DNA. The phage/retrovirus vector can be very useful for preparation of large expressing cDNA libraries with millions of recombinant clones. To increase the cloning capacity of the phage/virus vector a new modification was made of the original vector, named λgtLXL, which lacks selectable marker gene and has cloning limit up to 5 kb.

### Example 4. The use of tags to monitor negative selection of growth suppressive clones in retroviral library of fragmented p53

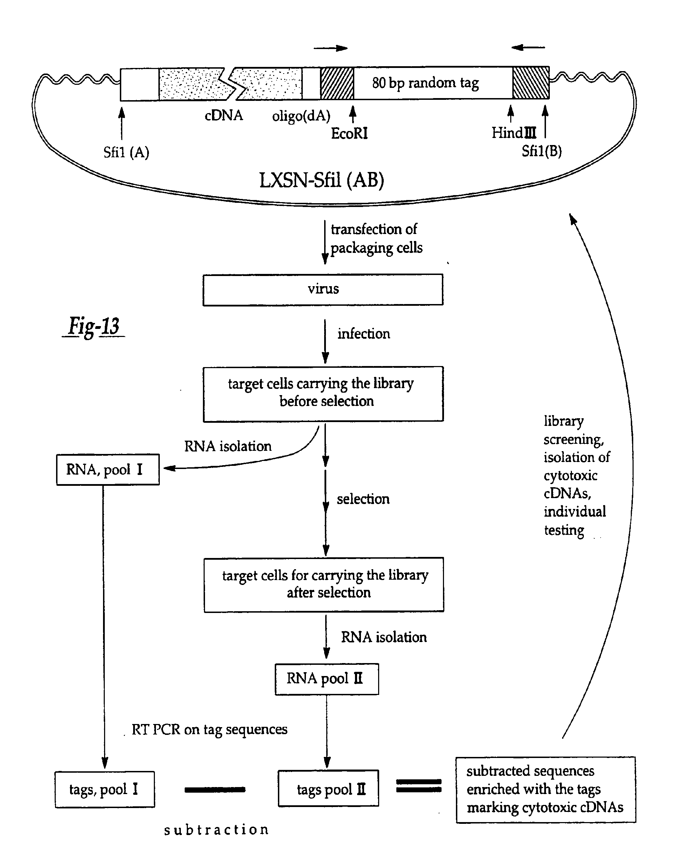

p53 is a key mediator of cell response to various types of stress. p53 gets activated after a variety of different stimuli (DNA damage, block of DNA replication, disruption of microtubules, cell detachment, hypoxia, etc.) and determines cell cycle arrest or apoptosis. To isolate the truncated growth suppressive version of p53 SSA was applied to screen the library of randomly fragmented p53 cDNA. The library was constructed as described above. Briefly, human p53 cDNA was partially digested by DNAse I in the presence of Mn⁺⁺ and the cDNA fragments ranging in size between 200 bp and full-length cDNA were ligated with two synthetic adaptors. One adaptor ("left") contained ATG codon in Kozak's consensus and EcoRI restriction site. A second adaptor ("right") contained stop codons in all reading frames and 80 bp random sequence ("tag") flanked by two 20-bp specific sequences for PCR amplification and BamHI restriction site (see Figures 4 and 12). After ligation with adaptors, the ligation mixture was digested by EcoRI and BamHI and cloned into a pLXSN retroviral vector expressing the inserts from the MoMuLV LTR promoter. The library of 30,000 independent clones was generated (Figure 13). The library was converted into a mixture of retroviral particles by transfection into BOSC23 packaging cells and the virus was used to infect several types of cells known to be sensitive to p53: HeLa cells (cervical carcinoma) expressing ecotropic viral receptor, mouse and human embryonic fibroblasts deficient in p53 (lines 10(1) and 041, respectively) and mouse embryonic fibroblasts transformed with Ela+ras (C8 cells) and highly sensitive to p53-dependent apoptosis. RNA was isolated (RNA pool 1) from each cell type 48 hours after infection and the remaining cells (50% of infected population) were maintained for one week after which cells were harvested for RNA isolation (RNA pool 2). RT PCR was performed on each RNA sample using primers for the sequences flanking 80-bp tags (the exact sequences of adaptors are shown in Figure 4). The resulting PCR products were used as a tester (tag pool 1) and driver (tag pool 2) DNAs for SSH subtraction procedure (Figure 10).

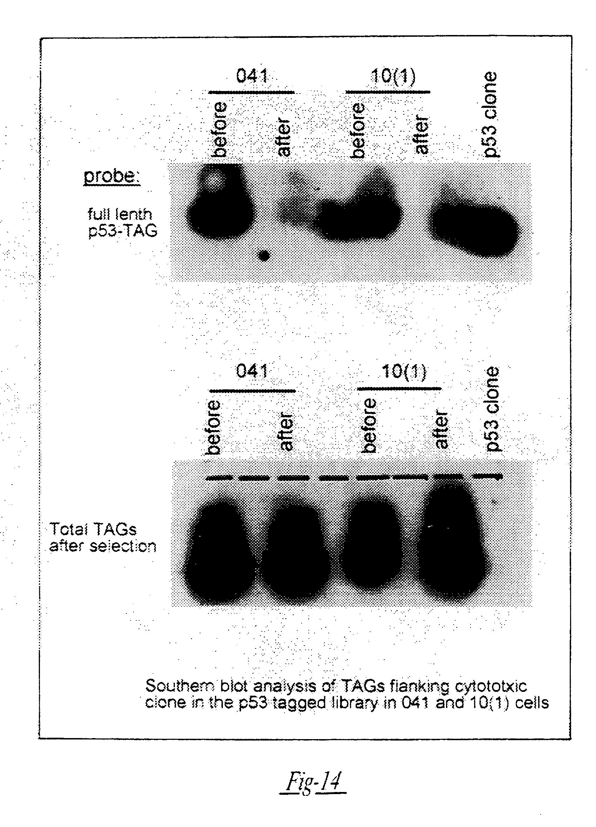

To insure that the loss of "toxic" clones occurs during selection, we isolated a clone from the library representing full-length p53 cDNA and used its tag as a probe for Southern hybridization to trace its behavior during selection. As it is shown in Figure 14, the hybridization signal of this clone is strongly decreased in both selected populations of target cells. These observations demonstrate that random 80 bp tag sequences can be used to monitor elimination of growth suppressive clones in tagged retroviral library delivered to target cells by hybridization.

### Example 5. Screening of tagged retroviral library of p53 cDNA fragments for growth suppressive clones

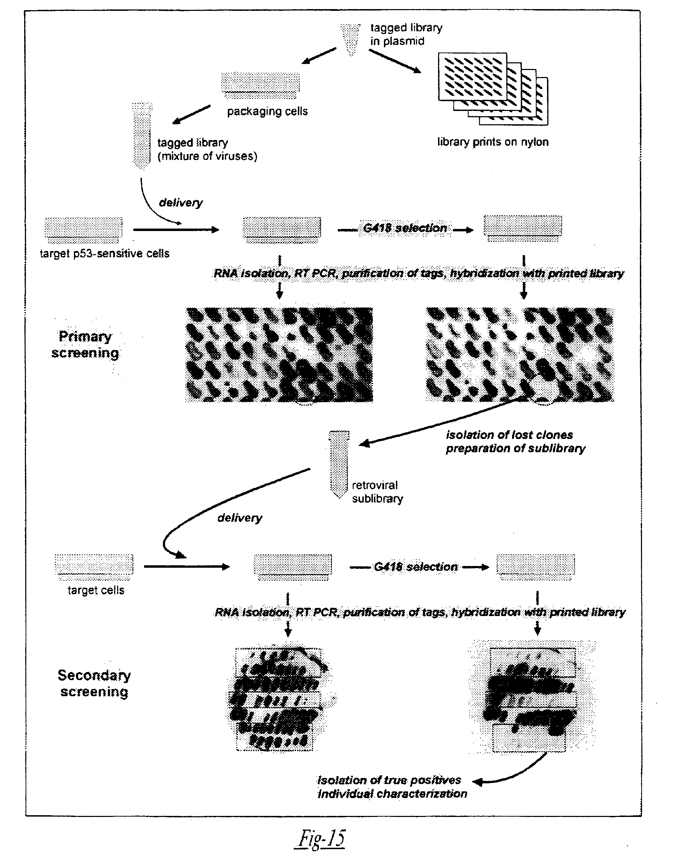

To imitate a microarray hybridization-based screening for differentially represented tags, a sublibrary of randomly picked 2,000 clones was separated from the tagged p53 library (see Example 4), all clones of which were printed onto nylon to form an array of clones for hybridization screening (see Figure 15). This mini-library was delivered to p53-sensitive 10(1) cells and tags were isolated by RT-PCR as described in Example 4 from cellular RNA isolated 24 hours after retrovirus infection and after completion of G418 selection (8 days after infection). Both isolates of tags were labeled with ³²P using a primer extension by Klenow fragment (tag adaptor sequences were used as primers) and the probes were hybridized separately with the library-carrying nylon membrane. Clones that demonstrated differential hybridization signal (low signal after G418 selection) were picked, grown and mixed together with several randomly picked control clones that did not show any changes after selection. This mixture was subjected to a second round of selection, tag recovery and hybridization. As it is shown in Figure 15, the majority of the clones originally picked for differential hybridization were confirmed in secondary screening. Seven such clones were isolated whose biological activity was individually confirmed by a direct transduction of target cells. p53 inserts were sequenced and were all found to contain full-length p53 cDNA. It is noteworthy that the proportion of full-length cDNA in the library was lower than 1% as judged by the results of PCR on individual randomly picked library clones.

These results indicate that identification and isolation of growth suppressive clones based on tag differential hybridization procedure can be done efficiently.

### Example 6. Monitoring the elimination of growth suppressive clones from cDNA retroviral library in different cell types

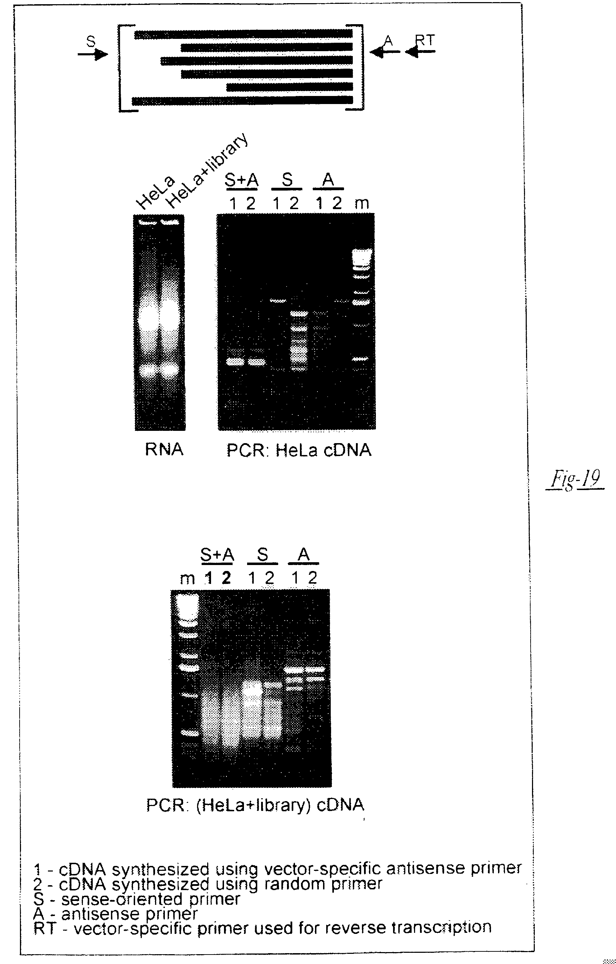

SSA methodology involves delivery of expression library into target cells, isolation of the inserts from the cells shortly after delivery (before selection) and after selection and identification of sequences that are differentially represented in two DNA populations recovered according to above described methods. However, the whole strategy would work only if the selection procedure applied is in fact sufficient to cause significant changes in the proportion of certain clones in the delivered library. To address this question, several retroviral cDNA libraries containing cDNA sequences encoding some known growth suppressors were delivered in several cell lines in order to set up the conditions of insert rescue and to monitor the fate of growth suppressive inserts during cell propagation. The results of these experiments are illustrated by Figures 19-21. Retroviral libraries of full-length cDNAs from HeLa cells were used, human placenta and normal human mammary gland manufactured by CLONTECH Laboratories. Retroviral vector pLIB used for the construction of these libraries contains no additional promoters or selectable markers to increase its cloning capacity. These libraries were converted from plasmid forms into mixtures of retroviruses by transfection of either ecotropic (BOSC23) or amphotropic (CAK8) packaging cells (Pear et al., 1993) and the resulting viruses were used to infect target cells. As target cells, human breast carcinoma cells MCF7 were used, human cervical carcinoma cell line HeLa and mouse embryo fibroblasts transformed by a combination of *E1a* and activated ras, line C8. Total RNA was isolated from part of the infected cells 24 hours after infection, while the rest of the infected population was maintained for additional 10 days and RNA was isolated at each passage of the infected cultures (1:3). cDNA was synthesized on all RNA preparations, using either vector-specific or random primers for reverse transcriptase. Library inserts were amplified from these cDNA samples by PCR with the primers corresponding to vector sequences flanking the inserts. Large number of primer combinations were tested to find the optimal pair that would allow to specifically amplify the inserts with no or low background on control cDNA synthesized using RNA from non-infected cells. The following pair of primers gave acceptable results (Figure 19) and therefore was chosen for further applications:
Sense primer (LXLS4): 5'-CCT TGA ACC TCC TCG TTC G-3'

Antisense primer (reverse 3) 5'-TTA CTT AAG CTA GCT TGC
CAA ACC-3'.

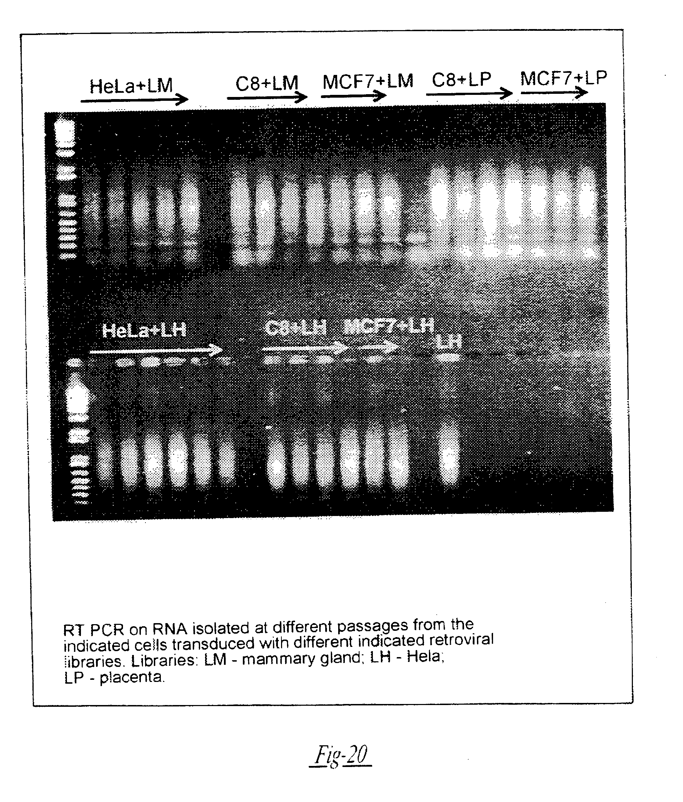

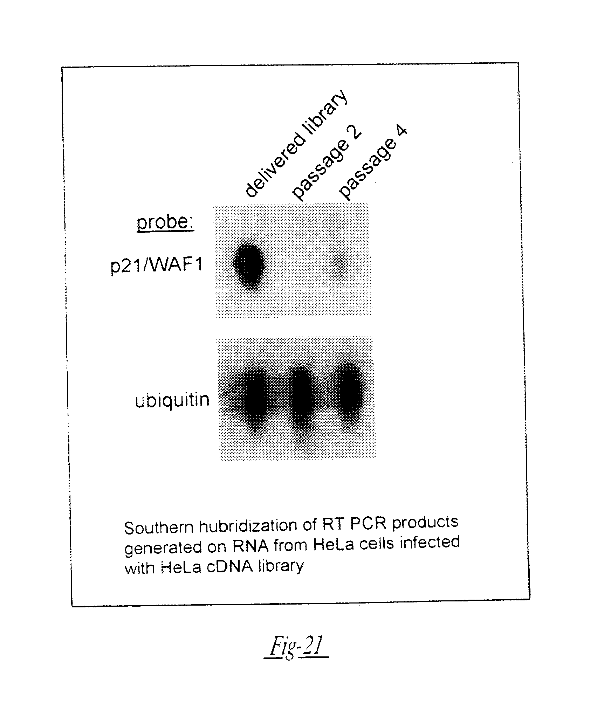

PCR products consisting of the recovered library inserts are shown in Figure 20. They were separated in agarose gels and analyzed by Southern hybridization. As probes for hybridization, the sequences encoding either non-toxic, non-cytostatic protein (ubiquitin) or known growth inhibitory protein (p21/WAF1) were used to monitor the presence and the proportion of clones with different biological activity during culture growth. The results of this analysis are shown in Figure 21. While the intensity of hybridization of ubiquitin probe does not change in passages, the proportion of p21/WAF1 sequences is significantly reduced consistently with the known growth suppressive effect of p21/WAF1 overexpression.

### Example 7. Isolation of tagged growth suppressive clones using RNAse H-based subtraction of tags

To set up the conditions allowing one to isolate growth suppressive clones from a retroviral expression library using tag-based subtraction selection approach (SSA) a tagged retroviral library of mouse embryonic cDNA cloned in modified pLXSN vector was used (see library description above). In order to be able to monitor the efficacy of the procedure of isolation of differentially represented tags, this library was mixed with a small amount (1/1,000) of a retroviral vector carrying poliovirus gene 2A tagged with a known 80 bp tag sequence. This construct had exactly the same design as any library-derived clone. Polioviral gene 2A encodes highly growth suppressive protein that is known to act as a potent inhibitor of cap-dependent translation. Strong biological effect of this vector was confirmed in gene transfer experiments (retrovirus transduction or transfection). A representative result of such testing is presented in Table 1.

**Table 1. G418 resistant colony formation by the indicated target cells infected or transfected by the indicated vectors. Numbers in parenthesis correspond to the proportion of positive cells during transient phase of transfection (monitoring by co-transfection with the GFP-expressing vector).**

| **Construct** | **Delivery** | **Cells** | | |
|---|---|---|---|---|
| | | **HeLa** | **10(1)** | **Rat1** |
| pLXSN (empty vector) | Transfection | 5,000 (20%) | 19,000 (12%) | 12,000 (17%) |
| | Infection (titer) | 110,000 | 180,000 | 210,000 |
| pL(2A)SN (carrying 2A gene) | Transfection | 0 (22%) | 0 (12%) | 4 (15%) |
| | Infection (titer) | 0 | 0 | 10 |

Tagged library including 0.1% of 2A-carrying clones was delivered to three different cell lines (Rat1 cells; Balb 3T3 cells, line 10(1) and human cervical carcinoma HeLa cells) by retrovirus transduction as described above. Cells were selected on G418. RNA was isolated from the cells 24 hours after library delivery and after G418 selection is completed and used for the RNAse H-based subtraction procedure. Tag sequences were isolated by RT-PCR from the post-selection pool of RNA. Universal primer-derived sequences were removed from tags by restriction digestion followed by gel purification.

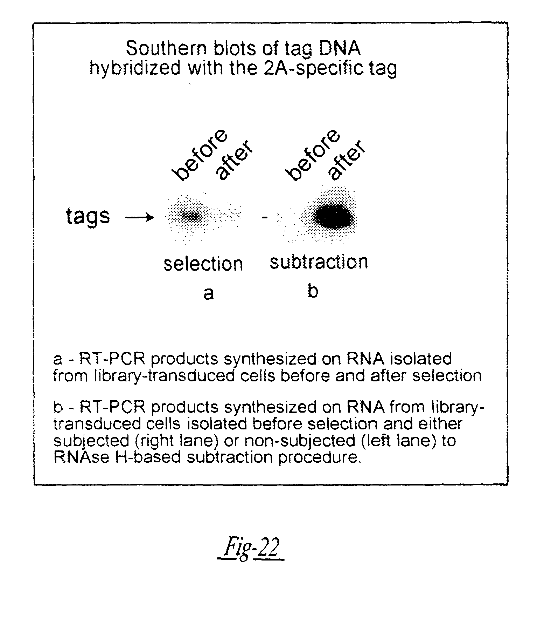

The resulting tag DNA was denatured and annealed with RNA prepared from the cells taken prior to selection as described in Methods section. After treatment with RNAse H, the remaining RNA was used for RT-PCR with vector-derived primers flanking tag sequences and with the primers allowing isolation of the complete insert (Figure 17). The presence of the specific tag, marking 2A-carrying clones, was determined by Southern hybridization in the original and subtracted tag populations (Figure 22). The obtained results show that RNAse H-based procedure resulted in a significant enrichment of the tags corresponding to the cytotoxic insert.

Throughout this application, various publications, are referenced by author and year. Full citations for the publications are listed below. The disclosures of these publications in their entireties are hereby incorporated by reference into this application in order to more fully describe the state of the art to which this invention pertains.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, the invention may be practiced otherwise than as specifically described.

### REFERENCES

Burke and Olson, "Preparation of Clone Libraries in Yeast Artificial-Chromosome Vectors" in Methods in Enzymology, Vol. 194, "Guide to Yeast Genetics and Molecular Biology", eds. C. Guthrie and G. Fink, Academic Press, Inc., Chap. 17, pp. 251-270 (1991).

Capecchi, "Altering the genome by homologous recombination" Science 244:1288-1292 (1989).

Cregg JM, Vedvick TS, Raschke WC: Recent Advances in the Expression of Foreign Genes in *Pichia pastoris,* Bio/Technology 11:905-910, 1993

Davies et al., "Targeted alterations in yeast artificial chromosomes for inter-species gene transfer", Nucleic Acids Research, Vol. 20, No. 11, pp. 2693-2698 (1992).

Diatchenko L, Lau YF, Campbell AP, Chenchik A, Moqadam F, Huang B, Lukyanov S, Lukyanov K, Gurskaya N, Sverdlov ED, Siebert PD (1996). Suppression subtractive hybridization: a method for generating differentially regulated or tissue-specific cDNA probes and libraries. Proc Natl Acad Sci U S A 93:6025-6030.

Dickinson et al., "High frequency gene targeting using insertional vectors", Human Molecular Genetics, Vol. 2, No. 8, pp. 1299-1302 (1993).

Duff and Lincoln, "Insertion of a pathogenic mutation into a yeast artificial chromosome containing the human APP gene and expression in ES cells", Research Advances in Alzheimer's Disease and Related Disorders, 1995.

Gilboa, E, Eglitis, MA, Kantoff, PW, Anderson, WF: Transfer and expression of cloned genes using retroviral vectors. BioTechniques 4(6):504-512, 1986.

Gudkov, A.V., Kazarov A.R., Thimmapaya, R., Mazo, I.A., Axenovich, S., and Roninson, I.B. (1994). Isolation of genetic suppressor elements from a retroviral normalized cDNA library: identification of a kinesin associated with drug sensitivity and senescence. *Proc. Natl. Acad. Sci. USA* **91**, 3744-3748.

Gudkov, A.V. and Roninson, I.B. (1996). Isolation of genetic suppressor elements (GSEs) from random fragment cDNA libraries in retroviral vectors. In: *Methods in Molecular Biology,* Vol. 32: *Protocols for cDNA Libraries.* Humana Press, Inc. Edited by: Ian Cowell & Caroline Austin. P. 221-240.

Huston et al, 1991 "Protein engineering of single-chain Fv analogs and fusion proteins" in Methods in Enzymology (JJ Langone, ed.; Academic Press, New York, NY) 203:46-88.

Huxley et al., "The human HPRT gene on a yeast artificial chromosome is functional when transferred to mouse cells by cell fusion", Genomics, 9:742-750 (1991).

Jakobovits et al., "Germ-line transmission and expression of a human-derived yeast artificial chromosome", Nature, Vol. 362, pp. 255-261 (1993).

Johnson and Bird, 1991 "Construction of single-chain Fvb derivatives of monoclonal antibodies and their production in *Escherichia coli* in Methods in Enzymology (JJ Langone, ed.; Academic Press, New York, NY) 203:88-99.

Lamb et al., "Introduction and expression of the 400 kilobase *precursor amyloid protein* gene in transgenic mice", Nature Genetics, Vol. 5, pp. 22-29 (1993).

Lisitsyn N, Lisitsyn N, Wigler M (1993). Cloning the differences between two complex genomes. Science Feb 12;259(5097):946-951.

Mernaugh and Mernaugh, 1995 "An overview of phage-displayed recombinant antibodies" in Molecular Methods In Plant Pathology (RP Singh and US Singh, eds.; CRC Press Inc., Boca Raton, FL) pp. 359-365.

Pearson and Choi, Expression of the human b-amyloid precursor protein gene from a heast artificial chromosome in transgenic mice. Proc. Natl. Scad. Sci. USA, 1993. 90:10578-82.

Rothstein, "Targeting, disruption, replacement, and allele rescue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301 (1991).

Russ AP, Friedel C, Grez M, von Melchner H (1996). Self-deleting retrovirus vectors for gene therapy. J Virol 70:4927-4932.

Schedl et al., "A yeast artificial chromosome covering the tyrosinase gene confers copy number-dependent expression in transgenic mice", Nature, Vol. 362, pp. 258-261 (1993).

Strauss et al., "Germ line transmission of a yeast artificial chromosome spanning the murine a₁ (I) collagen locus", Science, Vol. 259, pp. 1904-1907 (1993).

## Claims

1. A method of selection subtraction comprising the steps of:
a) creating an expression library wherein each clone is marked with a unique tag;
b) cloning a tagged clone produced in step (a) into a vector;
c) delivering the vector to target cells;
d) subjecting said cells to a selection procedure;
e) recovering tags from said cells before and after said selection procedure of step (d);
f) comparing differentially represented tags; and
g) using the tags to identify the desired clones which represent desired phenotypes.

2. A method according to claim 1, wherein step (f) comprises isolating genetic elements associated with complete or partial cell growth suppression or cell death, by isolating from the tagged expression library those clones that are lost in the cell population.

3. A method according to claim 1 for isolating genetic elements which induce any cell phenotype by isolating from an expression library clones which exhibit a predetermined selective trait, said method optionally further including isolating clones from an expression library which are associated with complete or partial growth suppression, by isolating from the expression library those clones that are lost in the cell population.

4. A method according to claim 2 or 3, wherein said isolating step further includes rescuing at least one clone from the cell before and after selection, said rescuing step preferably using PCR, RT-PCR, direct transformation, virus rescue and/or gene subtraction techniques.

5. A method of creating expression libraries by marking all clones with unique tags.

6. An expression library created according to any preceding claims consisting of individual clones marked with a unique tag sequence.

7. An expression library created according to claim 6, wherein said clones include:
a) an expressible sequence; and/or
b) mixtures of cDNAs cloned in sense and anti-sense orientation; and/or
c) mixtures of fragments of genomic DNA or cDNA; and/or
d) mixtures of artificial sequences.

8. A method according to claim 1, wherein step (f) comprises comparing differentially represented tags using hybridization on a DNA microarray.

9. A method of identification of differentially represented sequences in two populations of nucleic acids each marked with a unique tag derived from a clone wherein one is represented by RNA and another with DNA comprising the steps of:
a) annealing RNA and DNA sequences;
b) treatment with RNAseH or a similar enzyme destroying RNA within the RNA/DNA hybrid;
c) copying and amplifying remaining RNA molecules by polymerase reaction primed from sequences located outside of the specific RNA sequence.

10. A method of subtraction comprising the steps of:
a) subjecting cells including a unique tag derived from a clone to an RNAseH-based subtraction procedure, wherein each cell comprises a clone derived from an expression library, consisting of clones where each clone is marked with a unique tag;
b) recovering tags or inserts from cells before and after selection;
c) comparing differentially represented tags or inserts; and
d) using the tags or inserts to identify the desired clones which represent desired phenotypes.

11. A method of selection subtraction comprising the steps of:
a) creating an expression library with multiple clones wherein each clone is marked with a unique tag;
b) cloning a clone produced in step (a) into a vector;
c) delivering the vector to target cells;
d) subjecting said cells to a selection procedure;
e) recovering inserts from said cells before and after said selection procedure of step (d);
f) comparing differentially represented inserts; and
g) using the inserts to identify the desired clones which represent desired phenotypes.

12. A method according to claim 1, wherein step (f) comprises comparing pools of inserts using hybridization on a DNA microarray.

## Patentansprüche

1. Eine Methode der Selektionssubtraktion, die folgende Schritte umfasst:
a) Herstellung einer Expressionsbibliothek, wobei jeder Klon mit einem individuellen Tag markiert wird;
b) Klonieren eines in Schritt (a) hergestellten, getaggten Klons in einen Vektor;
c) Einbringen des Vektors in Zielzellen;
d) Die besagten Zellen einem Selektionsverfahren unterziehen;
e) Rückgewinnung der Tags aus den besagten Zellen vor und nach dem besagten Selektionsverfahren von Schritt (d);
f) Vergleich der unterschiedlich repräsentierten Tags und
g) Verwendung der Tags zur Identifizierung der gewünschten Klone, die die gewünschten Phänotypen repräsentieren.

2. Eine Methode gemäß Anspruch 1, wobei Schritt (f) die Isolierung genetischer Elemente umfasst, die mit vollständiger oder teilweiser Zellwachstumsunterdrückung oder Zelltod verbunden sind, indem von der getaggten Expressionsbibliothek solche Klone isoliert werden, die in der Zellpopulation verloren gegangen sind.

3. Eine Methode gemäß Anspruch 1 zur Isolierung genetischer Elemente, die einen Zellphänotypen induzieren, indem von einer Expressionsbibliothek Klone isoliert werden, die ein vorherbestimmtes selektives Merkmal aufweisen, wobei die besagte Methode wahlweise außerdem die Isolierung von Klonen aus einer Expressionsbibliothek umfasst, die mit vollständiger oder teilweiser Wachstumsunterdrückung verbunden sind, indem von der Expressionsbibliothek solche Klone isoliert werden, die in der Zellpopulation verloren gegangen sind.

4. Eine Methode gemäß Anspruch 2 oder 3, wobei der besagte Isolierungsschritt außerdem die Gewinnung mindestens eines Klons aus der Zelle vor und nach der Selektion umfasst, wobei bei dem besagten Gewinnungsschritt vorzugsweise PCR, RT-PCR, direkte Transformation, Virus-Rescue- und/oder Gensubtraktionstechniken verwendet werden.

5. Eine Methode der Herstellung von Expressionsbibliotheken durch Markierung aller Klone mit individuellen Tags.

6. Eine Expressionsbibliothek, die gemäß einem der vorstehenden Ansprüche hergestellt wird und die aus einzelnen Klonen besteht, die mit einer individuellen Tagsequenz markiert werden.

7. Eine Expressionsbibliothek, die gemäß Anspruch 6 hergestellt wird, wobei die besagten Klone Folgendes aufweisen:
a) eine exprimierbare Sequenz und/oder
b) Gemische aus cDNA, die in Sense- und Antisense-Orientierung geklont werden und/oder
c) Gemische aus Fragmenten von genomischer DNA oder cDNA und/oder
d) Gemische aus künstlichen Sequenzen.

8. Eine Methode gemäß Anspruch 1, wobei Schritt (f) den Vergleich von unterschiedlich repräsentierten Tags durch Hybridisierung auf einem DNA-Mikroarray umfasst.

9. Eine Methode zur Identifizierung von in zwei Populationen von Nukleinsäuren unterschiedlich repräsentierten Sequenzen, die mit einem individuellen, von einem Klon stammenden Tag markiert werden, wobei eine durch RNA und eine andere durch DNA repräsentiert wird, die die folgenden Schritte umfasst:
a) Annealing von RNA- und DNA-Sequenzen;
b) Behandlung mit RNAseH oder einem ähnlichen Enzym, das RNA innerhalb des RNA/DNA-Hybriden zerstört;
c) Kopieren und Amplifizieren der restlichen RNA-Moleküle durch Polymerasereaktion, die Sequenzen, die außerhalb der spezifischen RNA-Sequenz liegen, als Primer nutzt.

10. Eine Methode der Subtraktion, die folgende Schritte umfasst:
a) Zellen mit einem individuellen, von einem Klon stammenden Tag einem RNAseH-basierten Subtraktionsverfahren unterziehen, wobei jede Zelle einen Klon enthält, der aus einer Expressionsbibliothek stammt, die aus Klonen besteht, von denen jeder Klon mit einem individuellen Tag markiert wird;
b) Rückgewinnung der Tags oder Inserts aus den Zellen vor und nach der Selektion;
c) Vergleich von unterschiedlich repräsentierten Tags oder Inserts und
d) Verwendung der Tags oder Inserts zur Identifizierung der gewünschten Klone, die die gewünschten Phänotypen repräsentieren.

11. Eine Methode der Selektionssubtraktion, die folgende Schritte umfasst:
a) Herstellung einer Expressionsbibliothek mit Mehrfachklonen, wobei jeder Klon mit einem individuellen Tag markiert wird;
b) Klonen eines in Schritt (a) produzierten Klons in einen Vektor;
c) Einbringen des Vektors in Zielzellen;
d) Die besagten Zellen einem Selektionsverfahren unterziehen;
e) Rückgewinnung der Inserts aus den besagten Zellen vor und nach dem besagten Selektionsverfahren von Schritt (d);
f) Vergleich der unterschiedlich repräsentierten Inserts und
g) Verwendung der Inserts zur Identifizierung der gewünschten Klone, die die gewünschten Phänotypen repräsentieren.

12. Eine Methode gemäß Anspruch 1, wobei Schritt (f) den Vergleich von Pools von Inserts durch Hybridisierung auf einem DNA-Mikroarray umfasst.

## Revendications

1. Procédé de sélection-soustraction comprenant les étapes consistant à :
a) créer une banque d'expression dans laquelle chaque clone est marqué avec une étiquette unique ;
b) cloner un clone étiqueté produit dans l'étape (a) dans un vecteur ;
c) délivrer le vecteur à des cellules cibles ;
d) soumettre lesdites cellules à un mode opératoire de sélection ;
e) récupérer les étiquettes desdites cellules avant et après ledit mode opératoire de sélection de l'étape (d) ;
f) comparer les étiquettes représentées de manière différentielle ; et
g) utiliser les étiquettes pour identifier les clones souhaités qui représentent des phénotypes souhaités.

2. Procédé selon la revendication 1, dans lequel l'étape (f) comprend l'isolation d'éléments génétiques associés à une suppression de la croissance cellulaire complète ou partielle ou à une mort cellulaire, en isolant de la banque d'expression étiquetée ces clones qui sont perdus dans la population cellulaire.

3. Procédé selon la revendication 1 pour isoler des éléments génétiques qui induisent tout phénotype cellulaire en isolant d'une banque d'expression des clones qui présentent une caractéristique sélective prédéterminée, ledit procédé comprenant en outre éventuellement l'isolation de clones à partir d'une banque d'expression qui sont associés à une suppression de la croissance complète ou partielle, en isolant de la banque d'expression ces clones qui sont perdus dans la population cellulaire.

4. Procédé selon la revendication 2 ou 3, dans lequel ladite étape d'isolation comprend en outre le sauvetage d'au moins un clone de la cellule avant et après sélection, ladite étape de sauvetage utilisant de préférence une PCR, RT-PCR, une transformation directe, des techniques de sauvetage d'un virus et/ou de soustraction d'un gène.

5. Procédé de création de banques d'expression par marquage de tous les clones avec des étiquettes uniques.

6. Banque d'expression créée selon l'une quelconque des revendications précédentes consistant en des clones individuels marqués avec une séquence étiquette unique.

7. Banque d'expression créée selon la revendication 6, dans laquelle lesdits clones comprennent :
a) une séquence qui peut être exprimée ; et/ou
b) des mélanges d'ADNc clonés dans une orientation sens et anti-sens ; et/ou
c) des mélanges de fragments d'ADN génomique ou d'ADNc; et/ou
d) des mélanges de séquences artificielles.

8. Procédé selon la revendication 1, dans lequel l'étape (f) comprend la comparaison d'étiquettes représentées de manière différentielle en utilisant une hybridation sur un micro-réseau d'ADN.

9. Procédé d'identification de séquences représentées de manière différentielle dans deux populations d'acides nucléiques marquées chacune avec une étiquette unique dérivée d'un clone dans lequel l'une est représentée par de l'ARN et l'autre par de l'ADN comprenant les étapes consistant à :
a) anneler les séquences d'ARN et d'ADN;
b) traiter avec une RNAseH ou une enzyme similaire détruisant l'ARN dans l'hybride ARN/ADN;
c) copier et amplifier les molécules d'ARN restantes par réaction par polymérase amorcée à partir de séquences situées à l'extérieur de la séquence d'ARN spécifique.

10. Procédé de soustraction comprenant les étapes consistant à :
a) soumettre des cellules comprenant une étiquette unique dérivée d'un clone à un mode opératoire de soustraction à base de RNAseH, dans lequel chaque cellule comprend un clone dérivé d'une banque d'expression, consistant en des clones où chaque clone est marqué avec une étiquette unique ;
b) récupérer les étiquettes ou inserts à partir des cellules avant et après sélection;
c) comparer les étiquettes ou les inserts représentés de manière différentielle ; et
d) utiliser les étiquettes ou inserts pour identifier les clones souhaités qui représentent des phénotypes souhaités.

11. Procédé de sélection-soustraction comprenant les étapes consistant à :
a) créer une banque d'expression avec des clones multiples dans laquelle chaque clone est marqué avec une étiquette unique ;
b) cloner un clone produit dans l'étape (a) dans un vecteur ;
c) délivrer le vecteur à des cellules cibles;
d) soumettre lesdites cellules à un mode opératoire de sélection ;
e) récupérer les inserts desdites cellules avant et après ledit mode opératoire de sélection de l'étape (d) ;
f) comparer les inserts représentés de manière différentielle ; et
g) utiliser les inserts pour identifier les clones souhaités qui représentent des phénotypes souhaités.

12. Procédé selon la revendication 1, dans lequel l'étape (f) comprend la comparaison de groupes d'inserts en utilisant une hybridation sur un micro-réseau d'ADN.
